# EUROPÄISCHE PATENTSCHRIFT

(11) **EP 0 540 914 B1**
(45) Veröffentlichungstag und Bekanntmachung des Hinweises auf die Patenterteilung: **02.06.1999**
(21) Anmeldenummer: 92117605.3
(22) Anmeldetag: 15.10.1992
(51) Int. Cl.: C07D 311/58, C07D 311/64, C07D 405/12, C07D 407/12, C07D 493/04

(54) **2-Aminomethyl-chromane als Wirkstoffe in Arzneimitteln zur Behandlung von Erkrankungen des Zentralnervensystems**
2-Aminomethylchromans as active ingredients in medicaments for the treatment of diseases of the central nervous system
2-Aminométhylchromannes comme ingrédients actifs dans médicaments pour le traitement des maladies du système nerveux central

(30) Priorität: 28.10.1991 DE 4135474
(43) Veröffentlichungstag der Anmeldung: 12.05.1993
(73) Patentinhaber: BAYER AG, 51368 Leverkusen (DE)
(72) Erfinder: Schohe-Loop, Rudolf, Dr., W-5600 Wuppertal 11 (DE); Heine, Hans-Georg, Dr., W-4150 Krefeld (DE); Junge, Bodo, Dr., W-5600 Wuppertal 23 (DE); Glaser, Thomas, Dr., W-5063 Overath 3 (DE); De Vry, Jean Marie Viktor, Dr., W-5064 Rösrath (DE); Dompert, Wolfgang, Dr., W-5000 Köln 91 (DE); Sommermeyer, Henning, Dr., W-5000 Köln 80 (DE)

(56) Entgegenhaltungen:
- EP-A- 0 325 964
- EP-A- 0 352 613
- INDIAN JOURNAL OF CHEMISTRY, SECTION B, Bd. 20, Nr. 12, Dezember 1981, New Delhi, IN, Seiten 1063 - 1067 R. PRATAP, ET AL.: 'Phenoxyalkylamines in semirigid conformation: synthesis and pharmacological activity of N1-(4'-chromanyl)-N4-arylpiperazines and N 1-(2'-chromanylmethyl)-N4-arylpiperazines'

## Beschreibung

Die Erfindung betrifft 2-Aminomethyl-chromane, Verfahren zu ihrer Herstellung und ihre Verwendung in Arzneimitteln, insbesondere als Mittel zur Bekämpfung von Erkrankungen des zentralen Nervensystems.

Aus der DE 39 01 814 sind Aminomethyltetralin- und -chromanderivate mit einer ZNS-Aktivität bekannt. Außerdem werden in der DE 36 20 408 Aminomethyldihydrobenzopyranderivate beschrieben, die eine fungizide Wirkung besitzen. Ferner sind aus der EP 124 783 Benzofuran- und Benzopyrancarboxamide bekannt.

In EP-A-0 352 613 werden N-(1-Phenylethyl)-2-aminomethyl-chromane als synthetische Zwischenstufen erwähnt.

EP-A-0 325 964 beschreibt 4-Aminomethyl-chromane als Antagonisten des α-2-Adrenergen-Rezeptors.

Pratap et al., Ind. J. Chem. 1981, 20B, 1063 beschreibt N-[2-(4-Methoxyphenyl)ethyl]-2-aminomethylchroman mit hypotensiver Wirkung.

Die vorliegende Erfindung betrifft jetzt neue Aminomethyl-chromane der allgemeinen Formel (I) 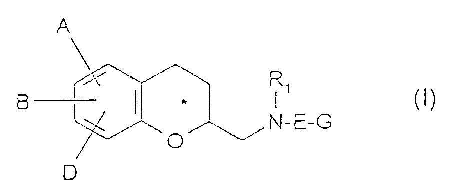 in welcher
- A, B und D: gleich oder verschieden sind und
für Wasserstoff, Fluor; Chlor; Brom, Cyano, Trifluormethyl, Difluormethoxy, Trifluormethoxy oder Hydroxy stehen,
für geradkettiges oder verzweigtes Alkyl, Alkenyl, Acyl oder Alkoxycarbonyl mit jeweils bis zu 6 Kohlenstoffatomen stehen,
für eine Gruppe der Formel -NR²R³, -NR⁴-L-R⁵ oder -OR⁶ stehen,
- R², R³ und R⁴: gleich oder verschieden sind und Wasserstoff oder geradkettiges oder verzweigtes Alkyl mit bis zu 6 Kohlenstoffatomen bedeuten,
- L: die -CO- oder -SO₂-Gruppe bedeutet
- R⁵: geradkettiges oder verzweigtes Alkyl mit bis zu 6 Kohlenstoffatomen oder Benzyl bedeutet, oder Phenyl bedeutet, das gegebenenfalls durch Fluor, Chlor, Brom, Trifluormethyl,Trifluormethoxy Hydroxy oder durch geradkettiges oder verzweigtes Alkyl oder Alkoxy mit jeweils bis zu 4 Kohlenstoffatomen substituiert ist,
- R⁶: geradkettiges oder verzweigtes Alkyl oder Alkenyl mit bis zu 6 Kohlenstoffatomen bedeutet, die gegebenenfalls durch Cyclopropyl, Cyclopentyl, Cyclohexyl oder Phenyl substituiert sind, oder
A eine der oben aufgeführten Bedeutungen hat und
B und D gemeinsam einen Rest der Formel 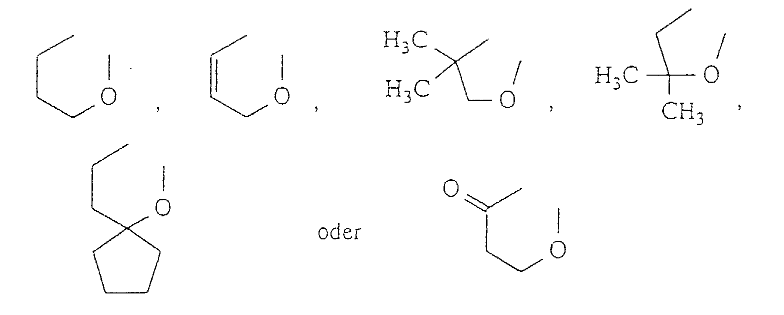 bilden,
E für eine direkte Bindung steht, oder
für geradkettiges oder verzweigtes Alkylen, Alkenylen oder Alkinylen mit jeweils bis zu 8 Kohlenstoffatomen steht, die gegebenenfalls durch Phenyl substituiert sind,
G für Phenyl, Naphthyl, Pyridyl, Chinolyl, Cyclopropyl, Cyclobutyl, Cyclopentyl, Cyclohexyl, Cycloheptyl, Cyclooctyl oder Adamantyl steht, die gegebenenfalls bis zu 2-fach gleich oder verschieden durch Hydroxy, Fluor, Chlor, Brom, Nitro, Cyano, Difluormethyl, Trifluormethyl, Difluormethoxy, Trifluormethoxy oder durch geradkettiges oder verzweigtes Alkyl oder Alkoxy mit jeweils bis zu 6 Kohlenstoffatomen substituiert sind, wobei letztere gegebenenfalls durch Phenyl oder Phenoxy substituiert sind, oder
für einen Rest der Formel 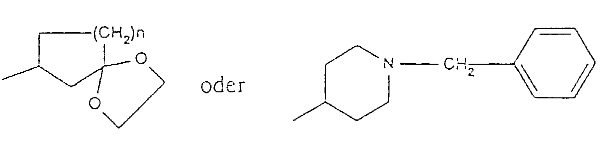 steht, worin
n eine Zahl 1 oder 2 bedeutet,
- R¹: für Wasserstoff,
für den Rest der Formel -E'-G' steht,
worin
E' und G' die oben für E und G angegebene Bedeutung haben und mit diesem gleich oder verschieden sind,
gegebenenfalls in einer isomeren Form,
und deren Salze,
wobei N-[1-Phenylethyl]-2-aminomethyl-chroman und
N-[1-phenylethyl]-2-aminomethyl-8-methoxy-chroman ausgenommen sind.

Im Rahmen der vorliegenden Erfindung werden physiologisch unbedenkliche Salze bevorzugt. Physiologisch unbedenkliche Salze der substituierten 2-Aminomethyl-chromane können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-. Kalium- und Ammoniumsalze.

Heterocyclus steht im allgemeinen für einen 5- bis 7-gliedrigen, bevorzugt 5- bis 6-gliedrigen, gesättigten oder ungesättigten Ring, der als Heteroatome bis zu 2 Sauerstoff- , Schwefel- und/oder Stickstoffatome enthalten kann. Bevorzugt sind 5-und 6-gliedrige Ringe mit einem Sauerstoff-, Schwefel und/oder bis zu 2 Stickstoffatomen. Bevorzugt werden genannt: Thienyl, Furyl, Pyrrolyl, Pyrazolyl, Pyranyl, Pyridyl, Pyrimidyl, Pyrazinyl, Pyridazinyl, Thiazolyl, Oxazolyl, Imidazolyl, Isoxazolyl, Pyrrolidinyl, Piperidinyl, Piperazinyl, Tetrazolyl, Morpholinyl oder Dioxanyl.

Verbrückter Bicarbocyclus steht im allgemeinen für 1-Adamantyl, 2-Adamantyl, Norbornyl, Bicyclo[2.2.3]octyl, Bicyclo[4.2.0]octyl oder Tetracyclo[5.2.2.0.0]undecanyl. Bevorzugt sind 1-Adamantyl, 2-Adamantyl oder Norbornyl.

Im Rahmen der vorliegenden Erfindung können die erfindungsgemäßen Verbindungen in verschiedenen stereoisomeren Formen vorliegen. Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher
- A, B und D: gleich oder verschieden sind und
für Wasserstoff, Fluor, Chlor, Brom, Cyano, Trifluormethyl, Trifluormethoxy oder Hydroxy stehen,
für geradkettiges oder verzweigtes Alkyl oder Alkenyl mit jeweils bis zu 4 Kohlenstoffatomen stehen,
für eine Gruppe der Formel -NR²R³ oder -OR⁶ stehen,
worin
R² und R³ gleich oder verschieden sind und Wasserstoff oder geradkettiges oder verzweigtes Alkyl mit bis zu 4 Kohlenstoffatomen bedeuten,
R⁶ geradkettiges oder verzweigtes Alkyl oder Alkenyl mit bis zu 4 Kohlenstoffatomen bedeutet, die gegebenenfalls durch Cyclopropyl oder Phenyl substituiert sind,
oder
- A: eine der oben aufgeführten Bedeutungen hat
und
- B und D: gemeinsam einen Rest der Formel 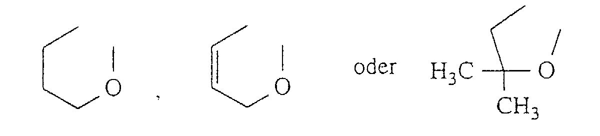 bilden,
- E: für eine direkte Bindung steht, oder
für geradkettiges oder verzweigtes Alkylen oder Alkenylen mit jeweils bis zu 7 Kohlenstoffatomen steht, die gegebenenfalls durch Phenyl substituiert sind,
- G: für Phenyl, Naphthyl, Adamantyl, Cyclopropyl, Cyclopentyl, Cyclohexyl, Cycloheptyl oder Cyclooctyl steht, die gegebenenfalls durch Fluor, Chlor, Brom, Hydroxy, Difluormethyl, Tnfluormethyl, Difluormethoxy, Trifluormethoxy oder durch geradkettiges oder verzweigtes Alkyl oder Alkoxy mit jeweils bis zu 4 Kohlenstoffatomen substituiert sind, wobei letztere gegebenenfalls durch Phenyl oder Phenoxy substituiert sind, oder
für einen Rest der Formel 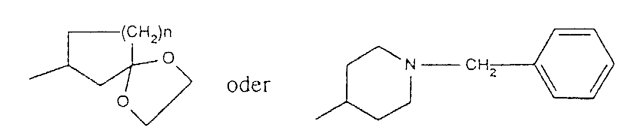 steht,
worin
n eine Zahl 1 oder 2 bedeutet,
und
- B und D: gemeinsam einen Rest der Formel 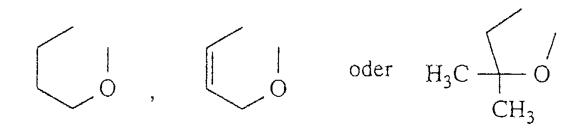 bilden,
- E: für eine direkte Bindung steht, oder
für geradkettiges oder verzweigtes Alkylen oder Alkenylen mit jeweils bis zu 7 Kohlenstoffatomen steht, die gegebenenfalls durch Phenyl substituiert sind,
- G: für Phenyl, Naphthyl Adamantyl, Cyclopropyl, Cyclopentyl, Cyclohexyl, Cycloheptyl oder Cyclooctyl steht, die gegebenenfalls durch Fluor, Chlor, Brom, Hydroxy, Difluormethyl, Trifluormethyl, Difluormethoxy, Trifluormethoxy oder durch geradkettiges oder verzweigtes Alkyl oder Alkoxy mit jeweils bis zu 4 Kohlenstoffatomen substituiert sind, wobei letztere gegebenenfalls durch Phenyl oder Phenoxy substituiert sind, oder
für einen Rest der Formel 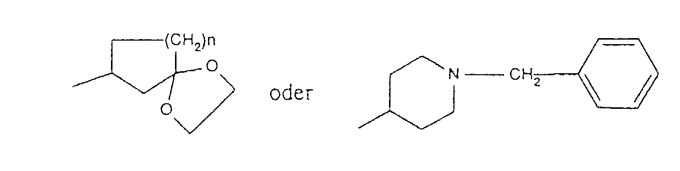 steht,
worin
n eine Zahl 1 oder 2 bedeutet,
R¹ für wasserstoff oder für den Rest der Formel -E'-G' steht,
worin
E' und G' die oben für E und G angegebene Bedeutung haben und mit diesen gleich oder verschieden sind, gegebenenfalls in einer isomeren Form,
und deren Salze, wobei N-[1-Phenylethyl]-2-aminomethylchroman und N-[1-phenylethyl]-2-aminomethyl-8-methoxychroman ausgenommen sind.

Außerdem wurden Verfahren zur Herstellung der erfindungsgemäßen Verbindungen der allgemeinen Formel (I) gefunden, dadurch gekennzeichnet, daß man
[A1] Verbindungen der allgemeinen Formel (II) 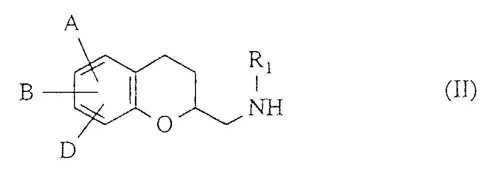 in welcher
   - A, B, D und R¹: die oben angegebene Bedeutung haben,
   mit Verbindungen der allgemeinen Formel (III)

   M-E-G (III)

   und
   - B und D: gemeinsam einen Rest der Formel 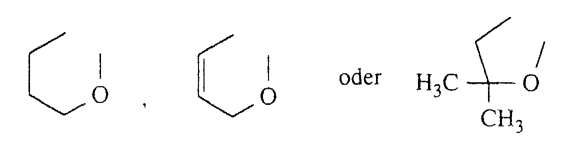 bilden,
   - E: für eine direkte Bindung steht, oder
   für geradkettiges oder verzweigtes Alkylen oder Alkenylen mit jeweils bis zu 7 Kohlenstoffatomen steht, die gegebenenfalls durch Phenyl substituiert sind,
   - G: für Phenyl, Naphthyl, Adamantyl, Cyclopropyl, Cyclopentyl, Cyclohexyl, Cycloheptyl oder Cyclooctyl steht, die gegebenenfalls durch Fluor, Chlor, Brom, Hydroxy, Difluormethyl, Trifluormethyl, Difluormethoxy, Trifluormethoxy oder durch geradkettiges oder verzweigtes Alkyl oder Alkoxy mit jeweils bis zu 4 Kohlenstoffatomen substituiert sind, wobei letztere gegebenenfalls durch Phenyl oder Phenoxy substituiert sind, oder
   für einen Rest der Formel 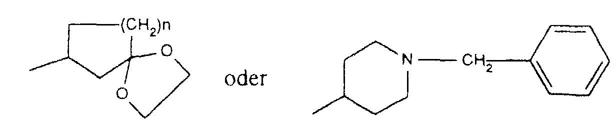 steht,
   worin
   n eine Zahl 1 oder 2 bedeutet,
   - R¹: für Wasserstoff oder für geradkettiges oder verzweiges Alkyl mit bis zu 4 Kohlenstoffatomen steht, oder
   für den Rest der Formel -E'-G' steht,
   worin
   E' und G' die oben für E und G angegebene Bedeutung haben und mit diesen gleich oder verschieden sind,
   gegebenenfalls in einer isomeren Form,
   und deren Salze.

Außerdem wurden Verfahren zur Herstellung der erfindungsgemäßen Verbindungen der allgemeinen Formel (I) gefunden, dadurch gekennzeichnet, daß man
[A1] Verbindungen der allgemeinen Formel (II) 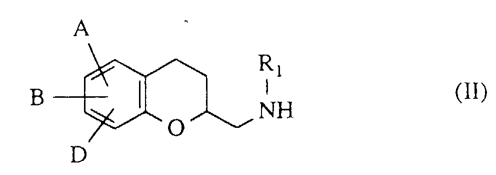 in welcher
   - A, B, D und R¹: die oben angegebene Bedeutung haben,
   mit Verbindungen der allgemeinen Formel (III)

   M-E-G (III)
   in welcher
   - M: für eine typische Abgangsgruppe wie Chlor, Brom, Iod, Tosylat, Mesylat oder für die Gruppe -OSO₂CF₃ steht,
   und
   - E und G: die oben angegebene Bedeutung haben,
   in inerten Lösemitteln, gegebenenfalls in Anwesenheit einer Base und/oder eines Katalysators alkyliert,
   oder
[A2] Verbindungen der allgemeinen Formel (IV) 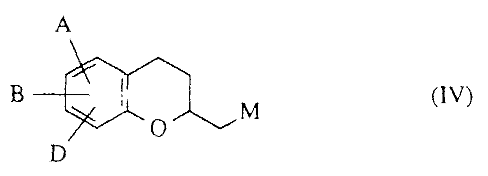 in welcher
   - A, B, D und M: die oben angegebene Bedeutung haben,
   mit Aminen der allgemeinen Formel (V) 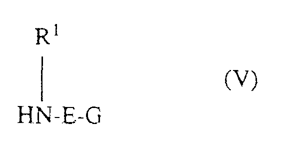
   in welcher
   - R¹, E und G: die oben angegebene Bedeutung haben,
   in inerten Lösemitteln, gegebenenfalls in Anwesenheit einer Base und/oder eines Katalysators alkyliert,
   oder
[B1] Aldehyde der allgemeinen Formel (VI) 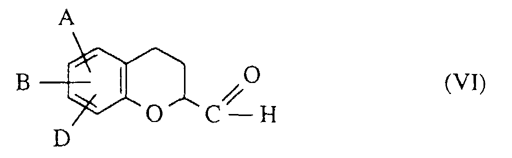 in welcher
   - A, B und D: die oben angegebene Bedeutung haben,
   mit Aminen der allgemeinen Formel (V)
   in inerten Lösemitteln, gegebenenfalls in Anwesenheit von Hilfsstoffen reduktiv alkyliert,
   oder
[B2] im Fall, daß E keine direkte Bindung bedeutet,
   Verbindungen der allgemeinen Formel (II) entweder mit Aldehyden der allgemeinen Formel (VII) 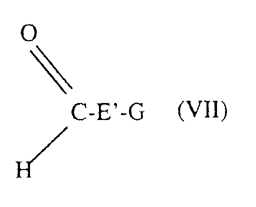 in welcher
   G die oben angegebene Bedeutung hat,
   E' die oben angegebene Bedeutung von E hat, aber um eine -CH₂-Gruppe verkürzt ist,
   oder im Fall, daß E für eine direkte Bindung steht, mit Verbindungen der allgemeinen Formel (VIIa)

   O=G' (VIIa)

   in welcher
   - G': die oben angegebene Bedeutung von G hat, aber nicht für Aryl steht,
   in inerten Lösemitteln, gegebenenfalls in Anwesenheit von Hilfsstoffen reduktiv alkyliert
   oder
[C] Verbindungen der allgemeinen Formeln (VIII) oder (IX) 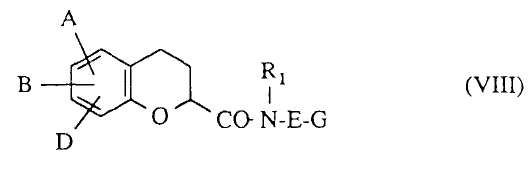 oder 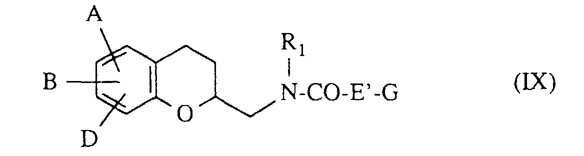 in welcher
   - A, B, D, E, E', G und R¹: die oben angegebene Bedeutung haben,
   in inerten Lösemitteln, gegebenenfalls in Anwesenheit von Hilfsstoffen, nach üblicher Methode reduziert,
   oder
[D] im Fall, daß E für 3 bis 6 Methylengruppen steht,
   Verbindungen der allgemeinen Formel (IIa) 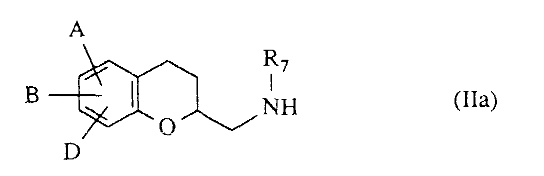 in welcher
   - A, B und D: die oben angegebene Bedeutung haben,
   und
   - R⁷: die oben angegebene Bedeutung von R¹ hat, aber nicht für Wasserstoff steht,
   zunächst entweder mit Formaldehyd oder Formaldehydderivaten und mit Verbindungen der allgemeinen Formel (X)

   E''-G (X)

   in welcher
   - G: die oben angegebene Bedeutung hat
   und
   - E'': für einen Rest der Formel HC≡C-(CH₂)_{z} steht,
   worin
   z eine Zahl 0, 1, 2 oder 3 bedeutet,
   in einer Mannichanalogen Reaktion umsetzt und gegebenenfalls in einem nächsten Schritt eine Hydrierung nach üblicher Methode anschließt,
   und im Fall, daß R¹ nicht für Wasserstoff steht, entweder nach üblicher Methode alkyliert oder mit Formaldehyd (R¹ = CH₃), wie oben beschrieben, reduktiv alkyliert,
   und im Fall, daß R¹ Wasserstoff bedeutet, gegebenenfalls während einzelner Verfahrensschritte zunächst die Aminfunktion mit geeigneten Aminoschutzgruppen blockiert und diese nach üblicher Methode, vorzugsweise durch Hydrogenolyse abspalten,
   und die Substituenten A, B, D und G nach üblicher Methode gegebenenfalls modifiziert.

Die erfindungsgemäßen Verfahren können durch folgendes Formelschema beispielhaft erläutert werden:

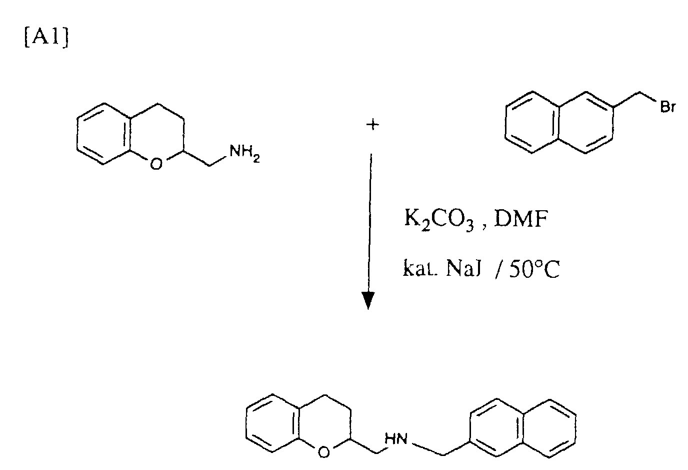

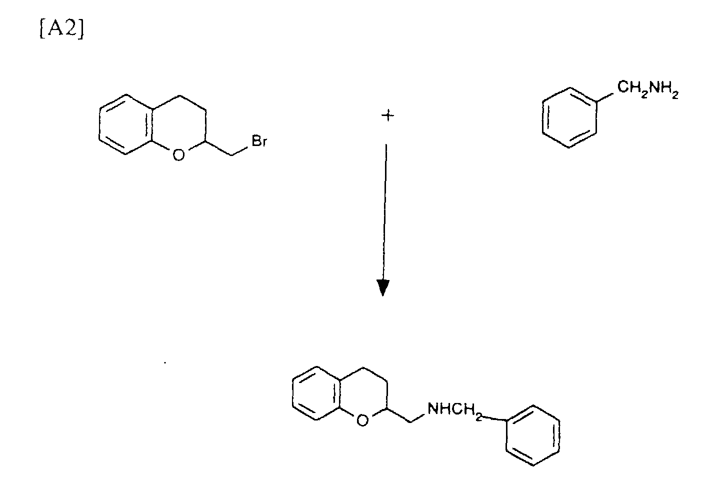

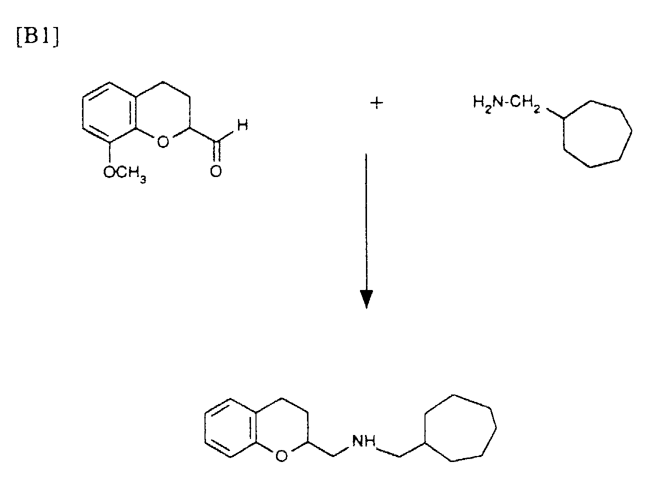

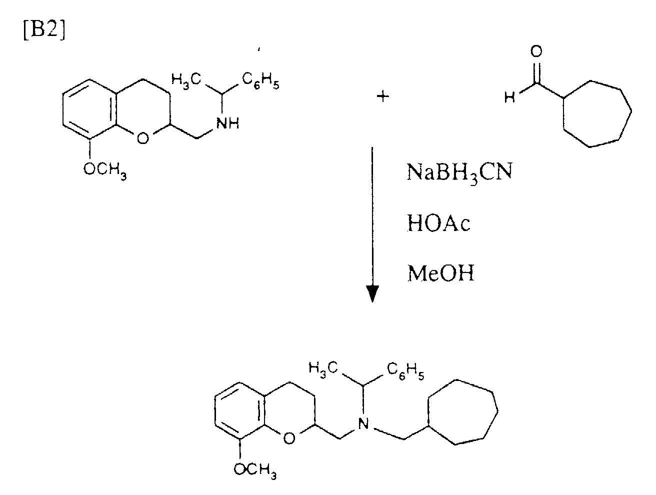

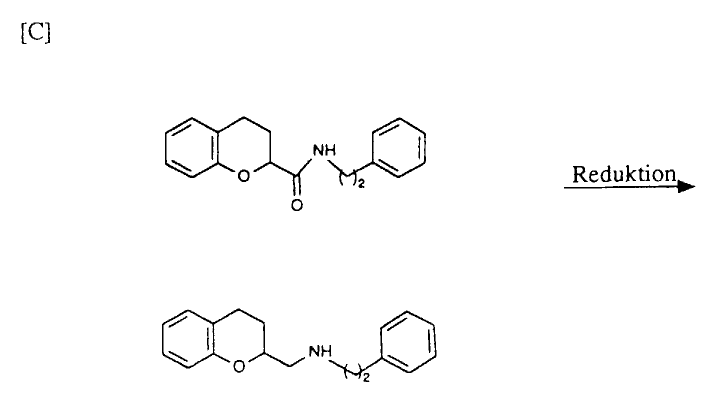

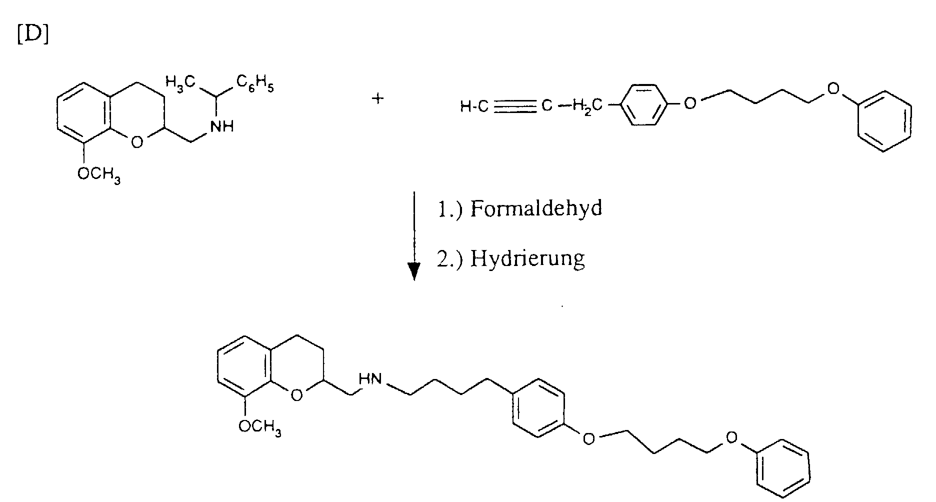

Für die Alkylierung eignen sich die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Butylmethylether, oder Ketone wie Aceton oder Butanon, oder Amide wie Dimethylformamid oder Hexamethylphosphorsäuretriamid, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel Gemische der genannten Lösemittel verwendet werden. Bevorzugt sind Methanol, Ethanol, Isopropanol, Dimethylformamid und Acetonitril.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Picolin oder N-Methylpiperidin, oder Amide wie Natriumamid oder Lithiumdiisopropylamid, oder metallorganische Verbindungen wie Butyllithium oder Phenyllithium. Bevorzugt sind Natrium- und Kaliumcarbonat, Pyridin und Triethylamin.

Die Alkylierung wird im allgemeinen in einem Temperaturbereich von 0°C bis +150°C, bevorzugt in einem Bereich von Raumtemperatur bis +80°C, durchgeführt.

Die Alkylierung wird im allgemeinen bei Normaldruck durchgeführt. Es ist jedoch ebenso möglich, die Reaktion bei erhöhtem oder erniedrigtem Druck durchzuführen.

Als Reaktionsbeschleuniger werden im allgemeinen Alkaliiodide eingesetzt, bevorzugt ist Natriumiodid oder Kaliumiodid.

Die Base wird hierbei in einer Menge von 1 bis 5, bevorzugt von 1 bis 2 Mol. bezogen auf 1 Mol der Verbindungen der allgemeinen Formeln (II), (IIa) und (IV) eingesetzt.

Die reduktive Aminierung der Amine der allgemeinen Formeln (II), (IIa) und (V) mit den Aldehyden der allgemeinen Formeln (VI) und (VII) und die reduktive Alkylierung der Verbindungen der allgemeinen Formeln (II) und (IIa) mit den Ketonen der allgemeinen Formel (VIIa) erfolgt im allgemeinen in einem Schritt. Im Fall eines primären Amins kann die Reaktion auch zweistufig durchgeführt werden, wobei zunächst eine Schiff'sche Base bzw. ein Enamin erhalten wird.

Die Herstellung der Schiff'schen Basen bzw. Enamine im ersten Schritt erfolgt in inerten organischen Lösemitteln, gegebenenfalls in Anwesenheit eines Katalysators und gegebenenfalls in Anwesenheit eines wasserbindenden Mittels. Das erfindungsgemäße Verfahren kann in 2 Schritten, d.h. mit Isolierung der Zwischenprodukte durchgeführt werden. Ebenso ist es möglich, die Reduktion als Eintopfverfahren durchzuführen.

Als inerte Lösemittel eignen sich hierbei die üblichen organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Butylmethylether, Dioxan, Tetrahydrofuran, Glykoldimethylether oder Diethylenglykoldiethylether, oder Halogenkohlenwasserstoffe wie beispielsweise Methylenchlorid, Chloroform oder Tetrachlorkohlenstoff, oder Kohlenwasserstoffe wie Benzol, Toluol, Xylol oder Erdölfraktionen, oder Amide wie Dimethylformamid oder Hexamethylphosphorsäuretriamid oder Essigsäure. Außerdem ist es möglich, Gemische der genannten Lösemittel zu verwenden. Bevorzugt sind Methanol, Ethanol, Diethylether, Tetrahydrofuran, Toluol und Chloroform.

Als Katalysatoren werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder mit Arylresten wie beispielsweise Methansulfonsäure, Ethansulfonsäure, Benzolsulfonsäure oder Toluolsulfonsäure.

Das bei der Reaktion gebildete Wasser kann gegebenenfalls im Gemisch mit dem verwendeten Lösemittel während oder nach der Reaktion z.B. durch Destillation oder durch Zugabe von wasserbindenden Mitteln, wie beispielsweise Phosporpentoxid oder bevorzugt durch Molekularsieb entfernt werden.

Die Reaktion wird im allgemeinen in einem Temperaturbereich von 0°C bis 150°C, bevorzugt von +20°C bis +100°C durchgeführt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Reduktion der Schiff'schen Basen bzw. Enamine im zweiten Schritt erfolgt entweder durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden in inerten Lösemitteln, gegebenenfalls in Anwesenheit eines Katalysators.

Bevorzugt wird die Reaktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Als Katalysatoren bei der Reduktion mit Natriumcyanoborhydrid werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten wie beispielsweise Methansulfonsäure, Ethansulfonsäure, Benzolsulfonsäure oder Toluolsulfonsäure.

Bei der Durchführung der erfindungsgemäßen Verfahren hat es sich als günstig erwiesen, die Umsetzung der Aldehyde (VI), (VII) und Ketone (VIIa) mit den Aminen (II), (IIa) und (V) in einem inerten Lösemittel, bevorzugt in Essigsäure oder Alkoholen wie beispielsweise Methanol, Ethanol, Propanol oder Isopropanol oder deren Gemischen, in Anwesenheit von anorganischen oder organischen Säuren wie beispielsweise Chlorwasserstoffsäure oder Essigsäure, und in Anwesenheit eines Reduktionsmittels, bevorzugt von komplexen Hydriden wie beispielsweise Natriumborhydrid oder Natriumcyanoborhydrid, gegebenenfalls in Anwesenheit eines wasserentziehenden Mittels, bevorzugt Molekularsieb, als Eintopfverfahren durchzuführen.

Im Falle des Einsatzes von Formaldehyd wird bevorzugt in mit Wasser mischbaren Lösemitteln wie Dioxan, Tetrahydrofuran, und mit phosphoriger Säure bzw. deren Salzen als Reduktionsmittel gearbeitet. Als Formaldehydquelle können auch wäßrige Formaldehydlösung oder Trioxan herangezogen werden. Das Reduktionsmittel wird equimolar zum Aldehyd eingesetzt.

Die Reduktion der Säureamide erfolgt entweder durch Wasserstoff in Wasser oder inerten organischen Lösemitteln wie Alkoholen, Ethern oder Halogenkotuenwasserstoffen oder deren Gemische, mit Katalysatoren wie Raney-Nickel, Palladium, Palladium auf Tierkohle oder Platin, oder aber mit Hydriden in inerten Lösemitteln, gegebenenfalls in Anwesenheit eines Katalysators, oder mit Boranen, Diboran oder ihren Komplexverbindungen.

Bevorzugt werden die Reaktionen mit Hydriden, wie komplexen Borhydriden, Aluminiumhydriden oder Natriumaluminiumdiethylhydrid, durchgeführt. Besonders bevorzugt werden hierbei Natrium-bis-(2-methoxyethoxy)dihydroaluminat, Lithiumaluminiumhydrid oder Diboran eingesetzt.

Die Umsetzung kann bei normalem, erhöhtem oder bei erniedrigtem Druck durchgeführt werden (z.B. 0,5 bis 150 bar). Im allgemeinen arbeitet man bei den Umsetzungen mit Hydriden und Boranen bei Normaldruck, bei den Umsetzungen mit Wasserstoff bei erhöhtem Druck.

Die Reduktion erfolgt im allgemeinen in einem Temperaturbereich von - 50°C bis zum jeweiligen Siedepunkt des Lösemittels, bevorzugt von -20°C bis +90°C.

Die Umsetzung mit Formaldehyd und Acetylenderivaten in einer Mannich-ähnlichen Reaktion wird im allgemeinen in einem der oben aufgeführten organischen Lösemittel, die sich unter den jeweiligen Reaktionsbedingungen nicht verändern, wie beispielsweise Alkohole, Ether, Kohlenwasserstoffe, Halogenkohlenwasserstoffe und Dimethylformamid sowie deren Gemische durchgeführt. Bevorzugt sind Tetrahydrofuran und 1,4-Dioxan.

Als Katalysatoren werden im allgemeinen Kupfersalze eingesetzt. Bevorzugt wird Kupfer(II)acetat. Als Formaldehydquelle gelangen Paraformaldehyd, Trioxan, Formalinlösung und gasförmiges Formaldehyd zum Einsatz. Bevorzugt wird Paraformaldehyd.

Die Reaktion erfolgt im allgemeinen in einem Temperaturbereich von 0°C bis Raumtemperatur, bevorzugt von +20°C bis +70°C.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Abspaltung der Aminoschutzgruppen erfolgt im allgemeinen mit Wasserstoff in Wasser oder einem der oben aufgeführten Lösemittel, vorzugsweise Wasser, Methanol, Ethanol, Diethylether, Tetrahydrofuran in Gegenwart von Mineralsäuren wie beispielsweise Salzsäure. Als Katalysatoren eignen sich die oben aufgeführten Katalysatoren, vorzugsweise Palladium und Palladium auf Tierkohle [vgl. Chem. and Biochemistry of the Amino Acids, G.C. Barrett, Chapman and Hall (1985)].

Der Katalysator wird in einer Menge von 0,01 Mol bis 0,2 Mol, bevorzugt von 0,05 Mol bis 0,15 Mol jeweils bezogen auf die blockierten Verbindungen der allgemeinen Formel (II) eingesetzt.

Die Umsetzung kann bei normalem, erhöhtem oder bei erniedrigtem Druck durchgeführt werden (z.B. 0,5 bis 25 bar). Im allgemeinen arbeitet man bei Normaldruck.

Die Reduktion erfolgt im allgemeinen in einem Temperaturbereich von - 50°C bis zum jeweiligen Siedepunkt des Lösemittels, bevorzugt von -20°C bis +90°C.

Die Abspaltung der Schutzgruppen aus den entsprechenden Ethern erfolgt nach üblicher Methode, beispielsweise durch Behandlung mit Protonensäuren wie Bromwasserstoff oder durch hydrogenolytische Spaltung der Benzylether in den oben aufgeführten inerten Lösemitteln in Anwesenheit eines Katalysators mit Waserstoff-Gas [vgl. außerdem Th. Greene: "Protective Groups in Organic Synthesis", J. Wiley/Sons, 1981, New York].

Die Verbindungen der allgemeinen Formeln (III), (V), (VII) und (VIIa) sind an sich bekannt oder können nach üblicher Methode hergestellt werden [vgl. Houben-Weyl "Methoden der organischen Chemie" Bd. XI/1 und XI/2, Beilstein 1, 594, 629, 662; Beilstein 2, 197, 201, 250, 278; 3, 9, 10, 21, 461, 462, 463].

Die Verbindungen der allgemeinen Formel (II) und (IIa) sind an sich bekannt [vgl. Indian J. Chem. Sect. B, 20B (12), 1063-7; DE 39 01 814] oder können nach üblicher Methode aus den entsprechenden Ketonen durch reduktive Aminierung, Alkylierung oder reduktive Alkylierung hergestellt werden.

Die Verbindungen der allgemeinen Formeln (IV) und (VI) sind an sich bekannt oder können nach üblicher Methode hergestellt werden [vgl. z.B. US 4 957 928; EP 252 005; EP 334 429; EP 145 067].
Die Verbindungen der allgemeinen Formel (VIII) sind teilweise bekannt [vgl. z.B.: US 4 238 506; DE 2 604 560], diejenigen der allgemeinen Formel (IX) dagegen neu und bilden sich bei der Umsetzung der jeweiligen Carbonsäuren, bzw. deren aktivierten Stufen, mit den Verbindungen der allgemeinen Formeln (II) oder (V).

Die Verbindungen der allgemeinen Formel (X) sind neu und können hergestellt werden, indem man im konkreten Fall beispielsweise 3-[4-(4-Phenoxybutoxy)phenyl]propin in 2 Stufen aus 4-Bromphenol durch Umsetzung mit 4-Brombutylphenylether in Gegenwart von Alkalicarbonat in Aceton herstellt und anschließend das Reaktionsprodukt mit Methoxyallen unter Cu(I)-Katalyse, umsetzt.

Die erfindungsgemäßen Verbindungen können als Wirkstoffe in Arzneimitteln verwendet werden. Die erfindungsgemäßen Stoffe haben eine besonders hohe Affinität zu cerebralen 5-Hydroxy-tryptamin-Rezeptoren vom 5-HT₁-Typ.

Sie haben agonistische, partiell agonistische oder antagonistische Wirkungen am Serotonin-Rezeptor. Im Vergleich zu strukturverwandten bekannten Verbindungen besitzen die erfindungsgemäßen Verbindungen überraschenderweise eine hohe Affinität zu Sigma-Rezeptoren.

Die in der vorliegenden Erfindung beschriebenen Verbindungen stellen somit Wirkstoffe zur Bekämpfung von Krankheiten dar, die durch Störungen des serotoninergen Systems, insbesondere bei Involvierung von Rezeptoren, die hohe Affinität zu 5-Hydroxytryptamin besitzen (5-HT₁-Typ), gekennzeichnet sind. Sie eignen sich daher zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuß- und Suchtmittelaufnahme. Weiterhin sind sie geeignet zur Beseitigung kognitiver Deficite, zur Verbesserung von Lern- und Gedächtnisleistungen und zur Behandlung der Alzheimer'schen Krankheit. Auch sind sie geeignet zur Bekämpfung von Psychosen (z.B- Schizophrenie, Manie). Gegenüber bekannter Neuroleptika besitzen sie ein geringeres Nebenwirkungspotential.

Weiterhin eignen sich diese Wirkstoffe auch zur Modulierung des cardiovaskulären Systems. Sie greifen auch in die Regulation der cerebralen Durchblutung ein und stellen somit wirkungsvolle Mittel zur Bekämpfung von Migräne dar.
Auch eignen sie sich zur Prophylaxe und Bekämpfung der Folgen cerebraler Infarktgeschehen (Apoplexia cerebri) wie Schlaganfall, cerebraler Ischämien. Darüberhinaus können die Verbindungen zur Behandlung von akutem Schädel-Him Trauma verwendet werden. Ebenso können die erfindungsgemäßen Verbindungen zur Bekämfpung von Schmerzzuständen eingesetzt werden. Auch eignen sie sich zur Bekämpfung von Störungen des Immunsystems.

### 1.) Affinität zum 5-HT₁-Rezeptor

In Tabelle A wird beispielhaft die hohe Affinität der erfindungsgemäßen Verbindungen zu 5-Hydroxytryptamin-Rezeptoren vom Subtyp 1 dargestellt. Bei den angegebenen Werten handelt es sich um Daten, die aus Rezeptorbindungsstudien mit Kalbs-Hippocampus-Membranpräparationen ermittelt wurden. Als radioaktiv markierter Ligand wurde hierzu ³H-Serotonin verwendet.

**Tabelle A**

| Verbindung des Beispiels | Kᵢ(nmol/l) |
|---|---|
| 23 | 5 |
| 13 | 36 |
| 18 | 3 |
| 22 | 6 |

### 2.) Affinität zum 5-HT_{1A}-Rezeptor [W.U. Dompert et al., Naunyn-Schmeideberg's Arch. Pharmacol. (1985), 328, 467-470].

Bei diesem Test wird die Bindung von ³H-Ipsapiron an 5-HT_{1A}-Rezeptoren in Kalbshippocampus-Membranen gemessen. Es wurde gefunden, daß die erfindungsgemäßen Verbindungen mit dem Radioliganden um die Bindung konkurrieren und diese hemmen.

**Tabelle B**

| Verbindung des Beispiels | Kiᵢ(nmol/l) |
|---|---|
| 17 | 0,7 |
| 28 | 2,3 |
| 31 | 4,2 |
| 35 | 28 |
| 36 | 0,5 |

### 3.) Affinität zum Sigma-Rezeptor

Der Sigmarezeptor-Bindungstest ist bei E. Weber et al. (1986), Proc. Natl. Acad. Sci. 83, 8784 - 8788 und M.P. Kavanaugh et al., (1988), Proc. Natl. Acad. Sci 85, 2844 im Detail beschrieben. Bei diesem Test wird die Bindung von 3-Di-o-tolyl-guanidin (DTG) an Kalbshippocampus-Membranen gemessen.

**Tabelle C**

| Verbindung des Beispiels | Kᵢ(nmol/l) |
|---|---|
| 1 | 7 |
| 23 | 30 |
| 13 | 5 |
| 17 | 8 |
| 31 | 16 |
| 36 | 25 |

Bei diesen Bindungstests werden IC₅₀-Werte ermittelt, die angeben, bei welcher Konzentration an Testsubstanz 50% der Bindung des Radioliganden verdrängt wird. Unter Berücksichtigung der Dissoziationskonstanten und der Konzentration an Radioliganden werden daraus die Inhibitionskonstanten Kᵢ berechnet.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Die jeweils aufgeführten R_{f}-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1%iger Kaliumpermanganat-Lösung.

Die Flash-Chromatographie wurde auf Kieselgel 60, 0,040 - 0,064 mm, Fa. E. Merck, durchgeführt (siehe Still et al., J. Org. Chem. 43, 2923, 1978; für einfachere Trennprobleme siehe Aldrichimica Acta 18, 25, 1985). Elution mit Lösemittelgradienten bedeutet: Beginnend mit der reinen, unpolaren Lösemittelgemischkomponente wird in einem steigendem Ausmaß die polare Laufmittelkomponente zugemischt, bis das gewünschte Produkt eluiert wird (DC-Kontrolle).

Bei allen Produkten wurde bei zuletzt ca. 0,1 Torr das Lösemittel abdestilliert. Salze wurden bei diesem Druck über Nacht über Kaliumhydroxid und/oder Phosphorpentoxid aufbewahrt.

### Lösemittelgemisch:

1 Dichlormethan / C₂H₅OH / NH₃ (200 : 10 : 1)
2 = Toluol / 2-Propanol (3 : 1 )
3 = Toluol / Essigester (3 : 1)
4 = Toluol / Essigester (1 : 1)

### Ausgangsverbindungen

### Beispiel I

N-(1(R)-Phenylethyl)-N-(2-(S)-chroman-2-yl-methyl)-cycloheptancarbonsäureamid 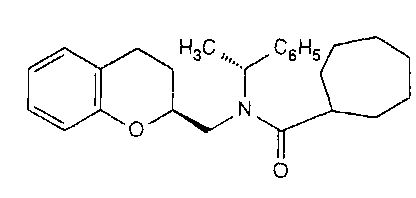 N-[1-(R)-phenylethyl]-2-(S)-aminomethylchroman (3,0 g; 11 mmol) (hergestellt in Analogie zu EP 352 613, Beispiel 92) wird in 33 ml Pyridin gelöst und mit 2,8 g (17 mmol) Cycloheptancarbonsäurechlorid versetzt. Nach 3 Stunden bei 50°C wird eingeengt und zwischen Wasser und Toluol verteilt. Filtration über Kieselgel und Einengen des Filtrats liefert das Amid als Rohprodukt, das weiter umgesetzt wird. R_{f} = 0,47 (Toluol/Essigester 3:1)

In Analogie zur Vorschrift des Beispiels I werden die in Tabelle I aufgeführten Verbindungen erhalten:

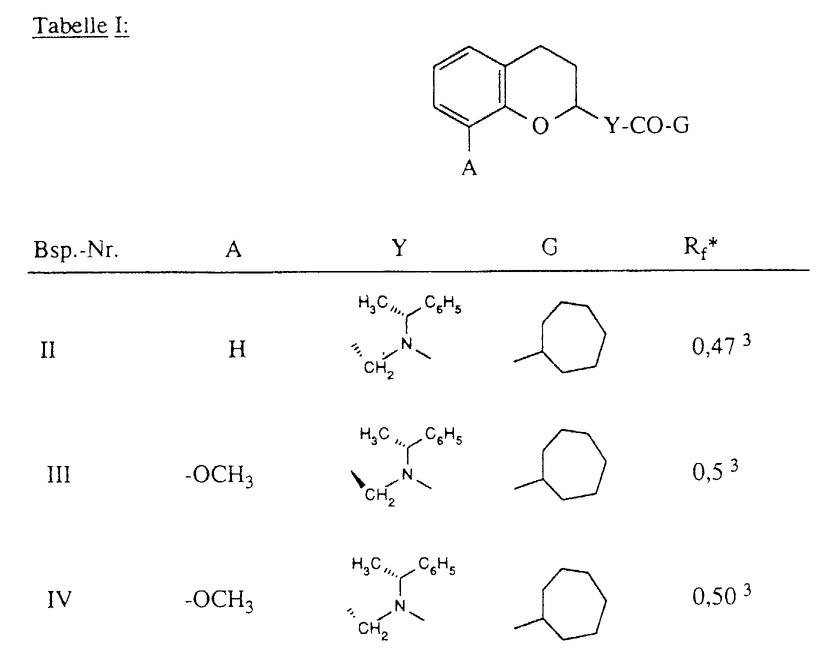

### Beispiel V

### N-(1-Naphthylmethyl)-chroman-2-carbonsäureamid

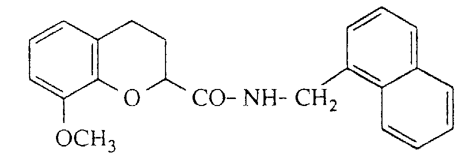

33,0 g (0,14 mol) 8-Methoxy-chroman-2-carbonsäureethylester (Gehalt 87%) werden zusammen mit 25,8 g (0,16 mol) 1-Aminomethylnaphthalin und 0,05 g Kaliumcyanid 8 Stunden auf 120°C und anschließend 2 Stunden auf 130°C erhitzt. Nach Abkühlen wird mit 100 ml Toluol verdünnt und diese Lösung auf Kieselgel K₆₀ gegeben. Elution mit Cyclohexan-Essigester-Gemischen (100:0 bis 75:25) liefert das Amid in Form eins zähen Sirups (32,9 g, 68%), das direkt weiter umgesetzt werden kann.

In Analogie zur Vorschrift des Beispiels V werden die in Tabelle II aufgeführten Verbindungen hergestellt:

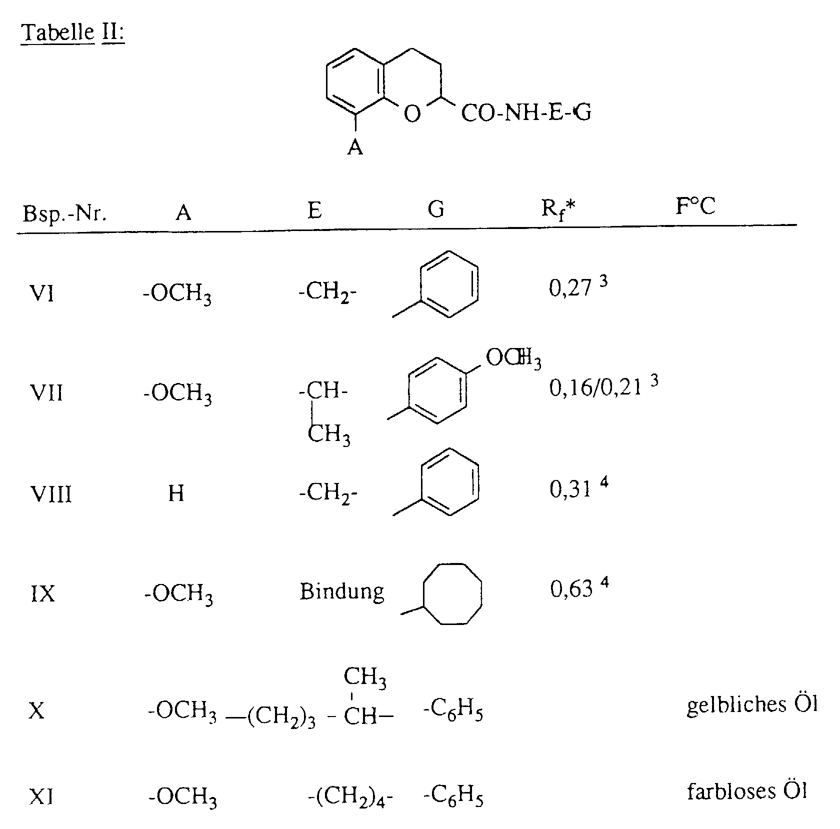

### Beispiel XII

N-(Chroman-2-yl)methyl-cycloheptancarbonsäureamid 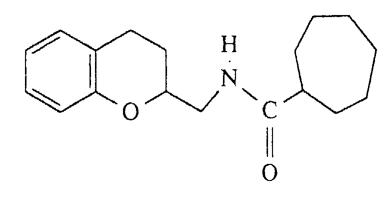 5,0 g (25 mmol) 2-Aminomethylchroman-Hydrochlorid werden in 50 ml Pyridin gelöst; hierzu werden 5,6 g (35 mmol) Cycloheptancarbonsäurechlorid zugetropft. Nach 1 Stunde bei 50°C wird eingeengt und der Rückstand zwischen Wasser und Toluol/Essigester 1:1 verteilt. Die organische Phase wird über Kieselgel filtriert Das gewünschte Produkt wird mit Toluol/Essigester 5:1 eluiert. Das nach Einengen erhaltene Amid wird direkt weiter umgesetzt.

### Beispiel XIII

N-(8-Methoxy-chroman-2-yl)methyl-cycloheptancarbonsäureamid

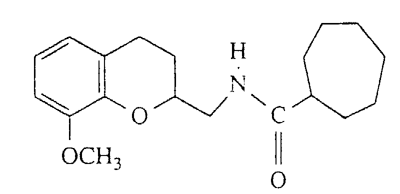

Die Titelverbindung wird in Analogie zur Vorschrift des Beispiels XII aus 2-Aminomethyl-8-methoxychroman-Hydrochlorid und Cycloheptancarbonsäurechlorid hergestellt, und direkt weiter umgesetzt.
F°C: 135

### Beispiel XIV

2-(R)-2-{N-([1-(R)-1-Phenethyl]-N-[4-[4-(4-Phenoxybutyloxy)-phenyl]-butyl}]aminomethyl-8-methoxy-chroman Hydrochlorid

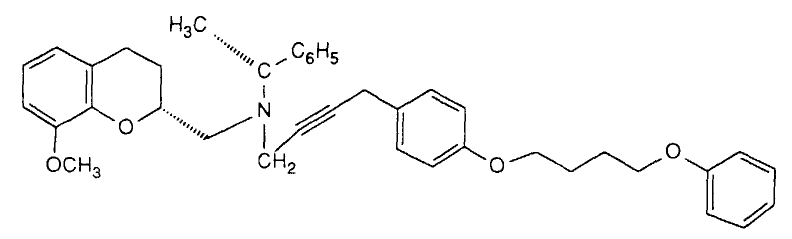

Das analog zu EP 352 613 / Beispiel 93 aus (R)-Phenylethylamin erhältliche 2-(R)-2-{N-[1-(R)-Phenethyl]}-aminomethyl-8-methoxy-chroman wird mit 3-[4-(4-Phenoxybutoxy)phenyl]-propin und Paraformaldehyd in Gegenwart von Cu(II)-Acetat in Dioxan bei 50°C umgesetzt. Nach chromatographischer Reinigung und Fällung des Hydrochlorids erhält man das gewünschte Zwischenprodukt in 63% Ausbeute als farblosen, amorphen Feststoff.
R_{f} = 0.14 (Toluol/Petrolether 1:1)

### Beispiel XV

(2S)-2-[N-Cycloheptylmethyl-N-((1R)-1-phenethyl)]aminomethyl-chroman Hydrochlorid

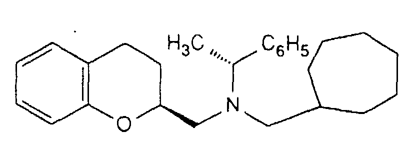

4,3 g der Verbindung aus Beispiel I werden in 25 ml Toluol aufgenommen und mit 22 g einer 3,4 M Lösung von Natrium-bis-(2-methoxyethoxy)-dihydroaluminat in Toluol versetzt. Nach 1 h Rühren bei 50°C wird nach Abkühlen auf Raumtemperatur mit 50 ml Toluol verdünnt und mit ca. 5 ml Wasser bis zum Ende der Gasentwicklung versetzt. Das Reaktionsgemisch wird über Kieselgel filtriert (Nachwaschen mit Toluol/Essigester 30:1). Die nach Einengen der produkthaltigen Fraktionen erhaltene freie Base wird mit etherischer Salzsäure in das Hydrochlorid überführt.
R_{F} = 0,92 (Toluol / Essigester 3:1)

In Analogie zur Vorschrift des Beispiels XV werden die in Tabelle III aufgeführten Verbindungen hergestellt:

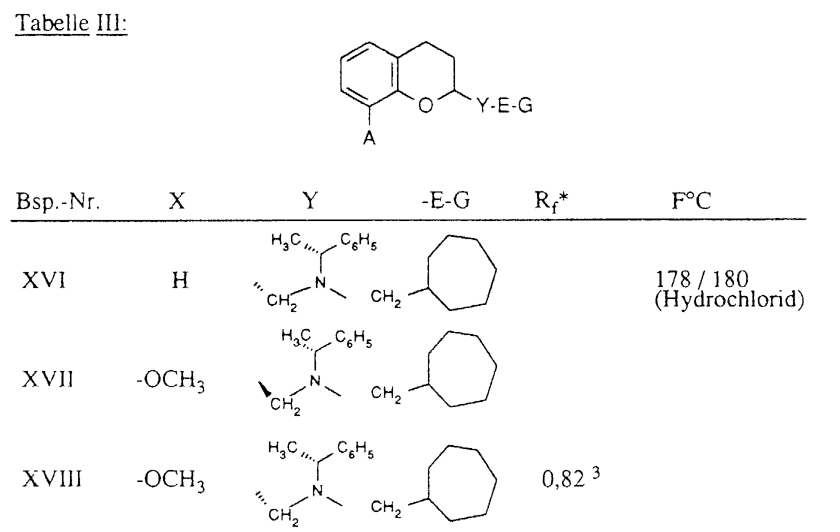

### Beispiel XIX

N-(8-Methoxychroman-2-yl-methyl)-cyclohexancarbonsäureamid

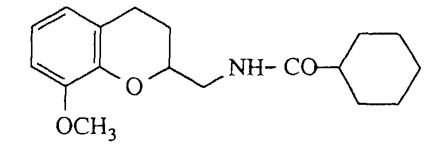

Zu der Lösung von 7,2 g (49 mmol) Cyclohexancarbonsäurechlorid in 75 ml wasserfreiem Dichlormethan werden unter Rühren und Eiskühlung das Gemisch aus 9,5 g (49 mmol) 2-Aminomethyl-8-methoxychroman und 4,9 ml (49 mmol) Triethylamin in 50 ml wasserfreiem Dichlormethan getropft. Nach Stehenlassen über Nacht wird der Ansatz aus Eis gegossen. Trennen der Phasen, Extrahieren der wäßrigen Phase mit Dichlormethan, Waschen mit gesättigter Kochsalzlösung und Trocknen der vereinigten organischen Phasen über wasserfreiem Natriumsulfat liefern nach Eindampfen im Wasserstrahlvakuum 14,5 g rohes Amid, das aus 160 ml Essigsäureethylester umkristallisiert wird.
IR (KBr). 3307, 2931, 1642, 1485 und 1256 cm
Fp.: 149-151°C

In Analogie zur Vorschrift des Beispiels XIX werden die in Tabelle IV, V, VI und VII aufgeführten Verbindungen hergestellt:

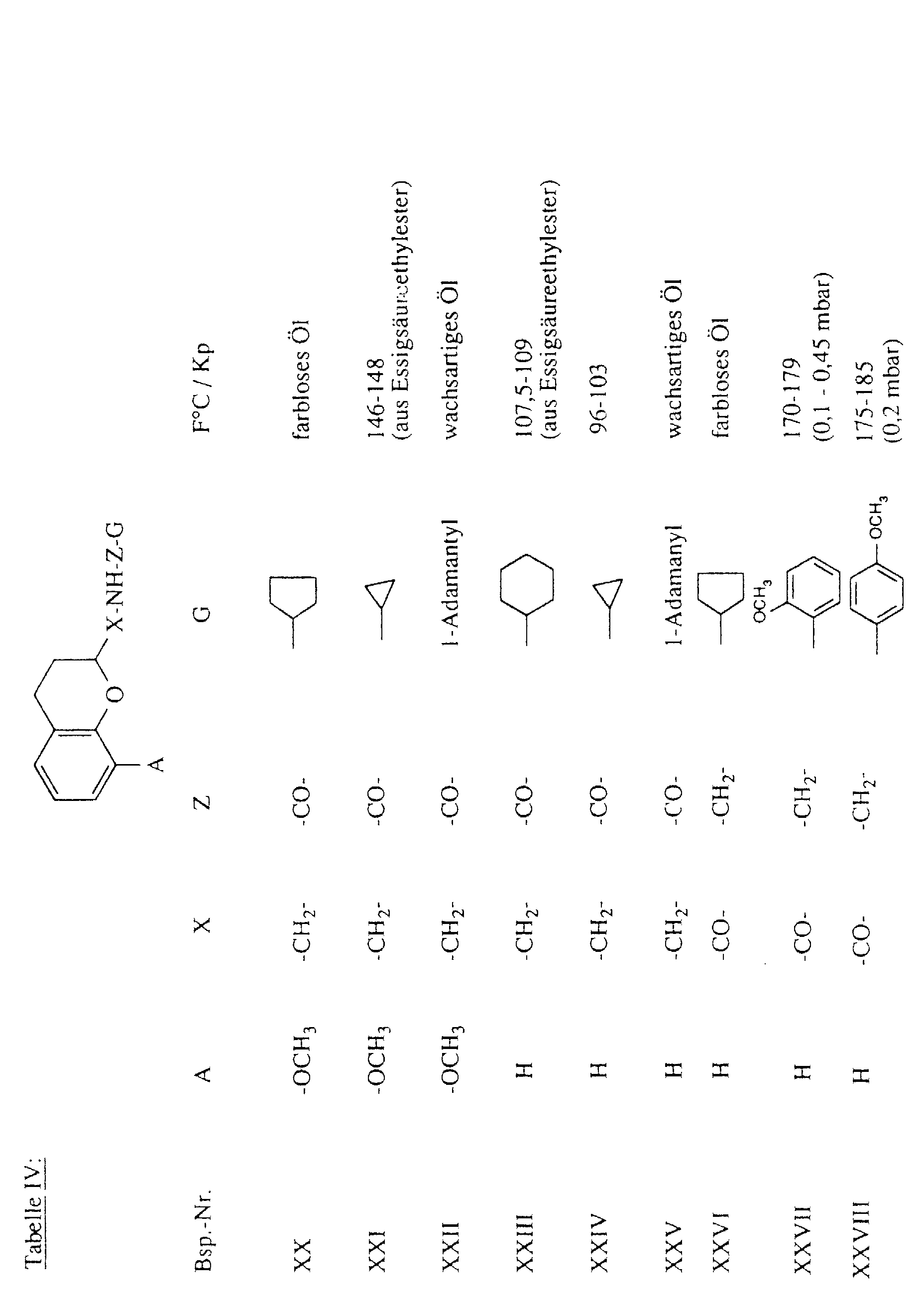

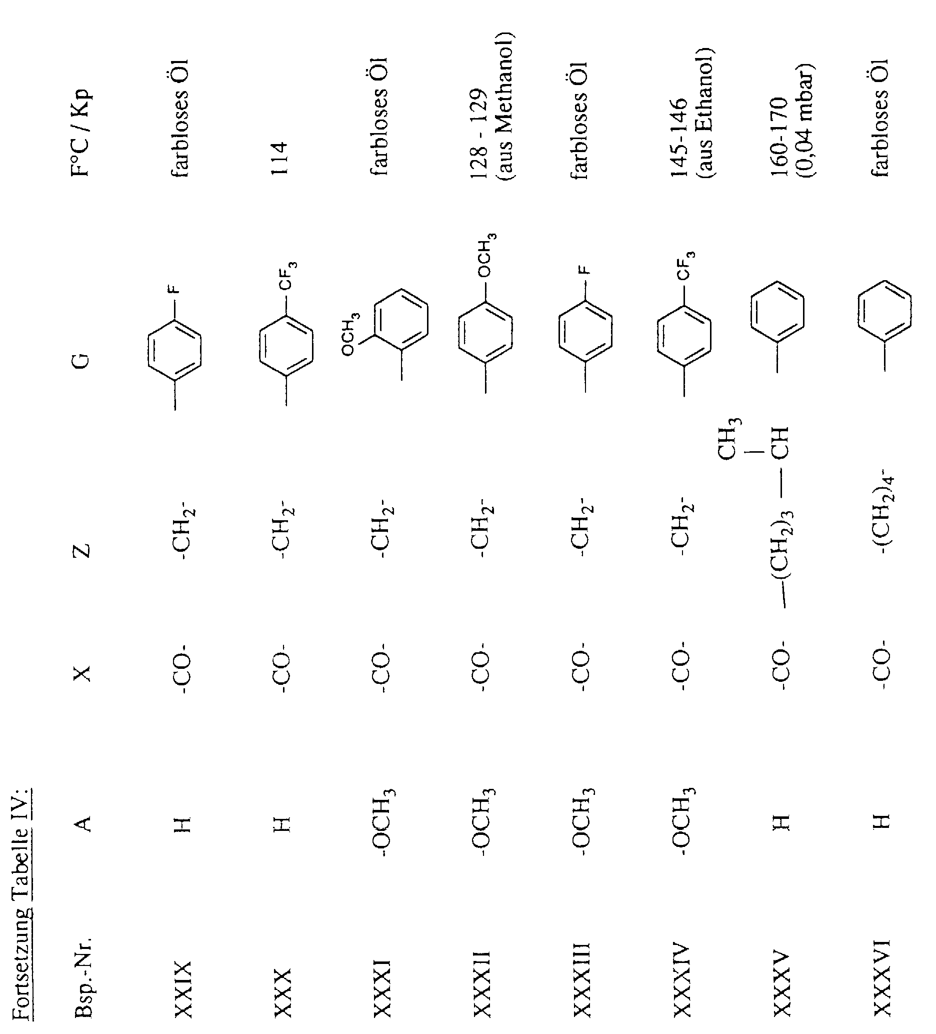

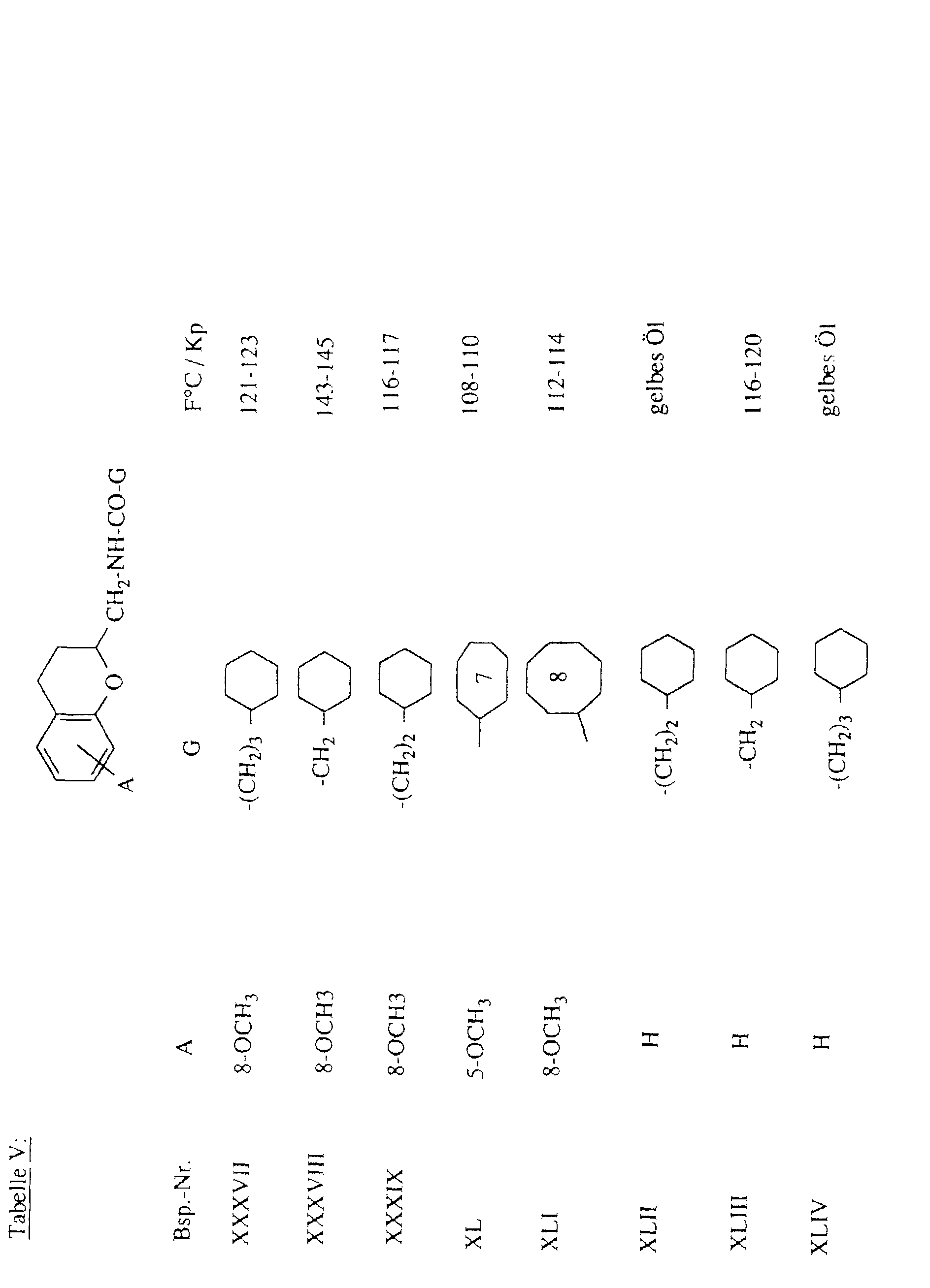

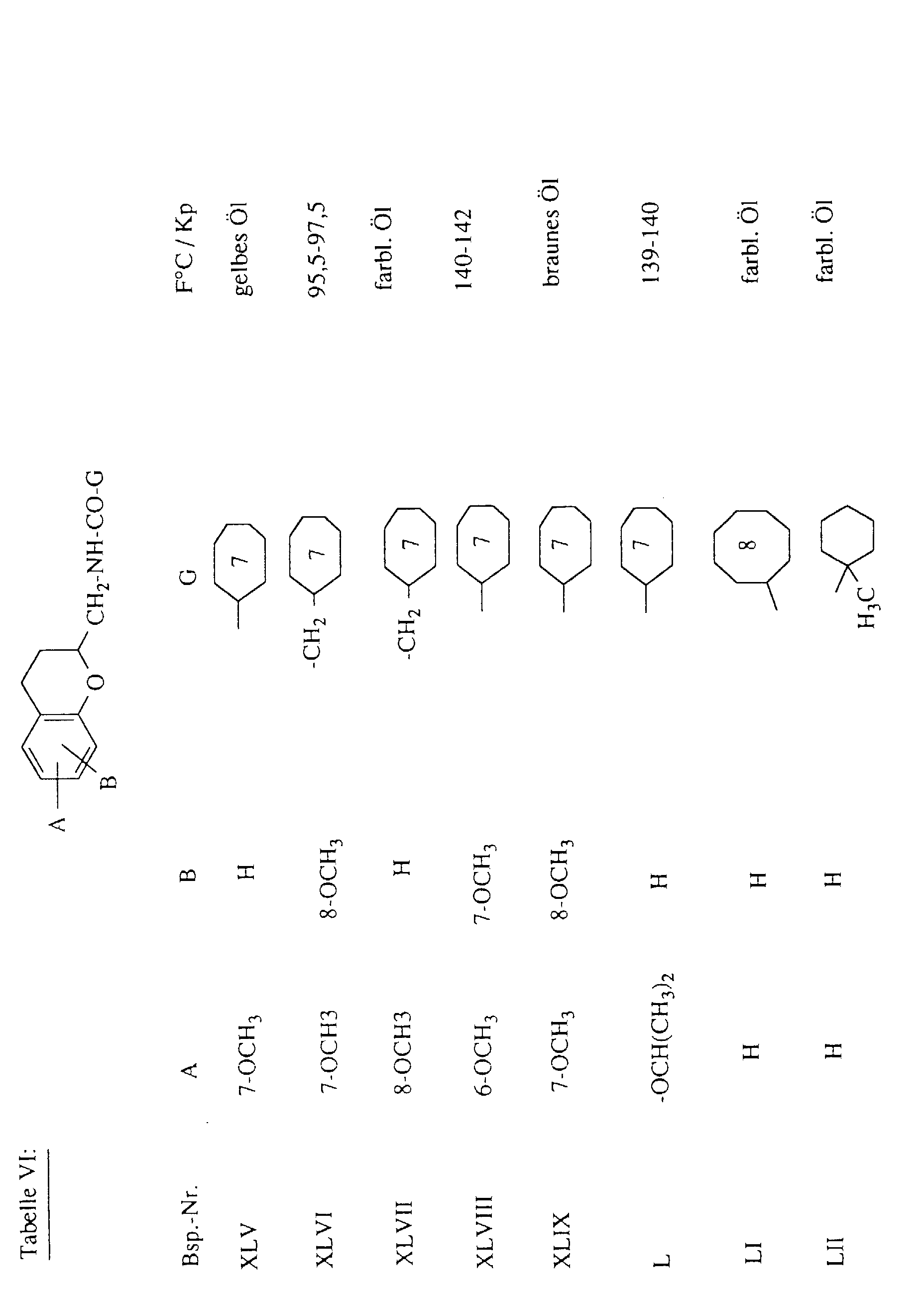

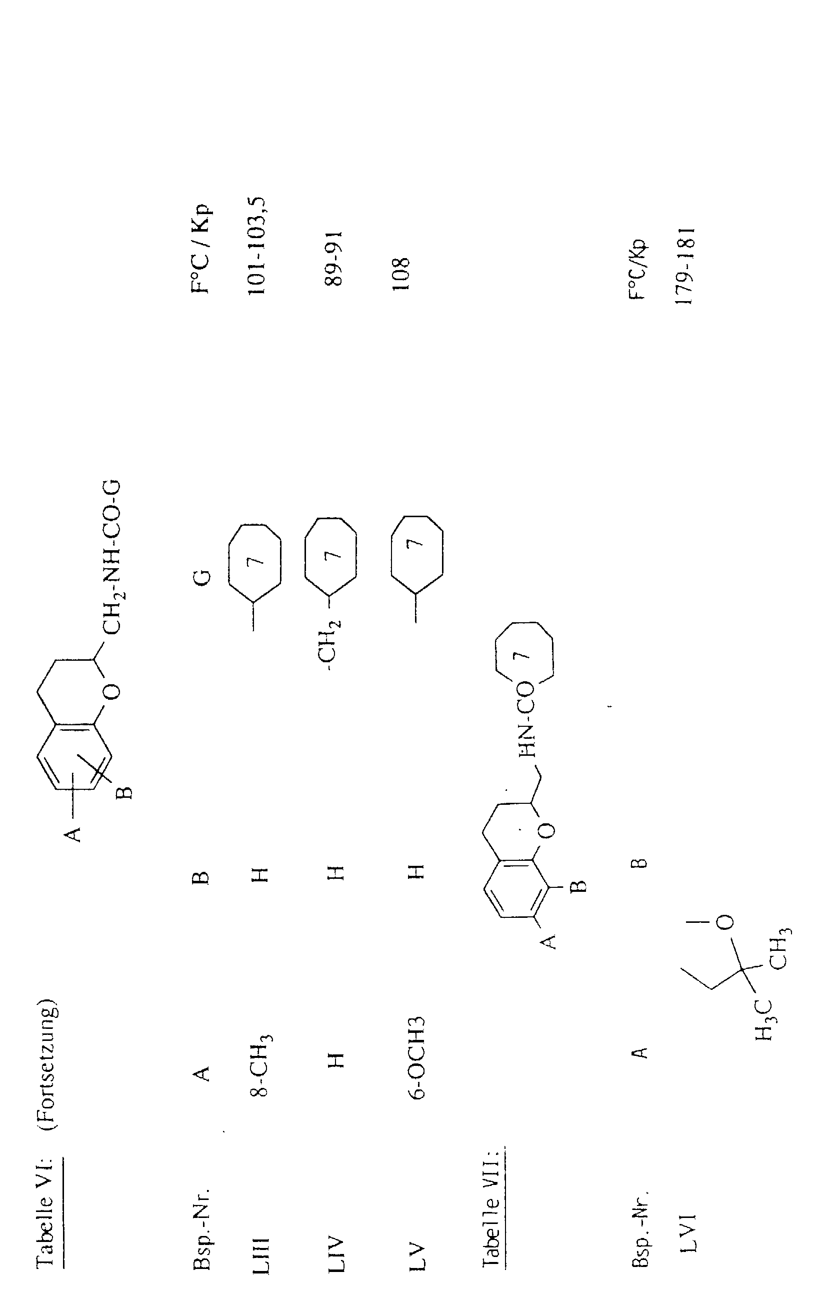

### Beispiel LVII

2-(N-Benzylpiperidin-4-carbonyl)-aminomethyl-3,4-dihydro-2H-1-benzopyran

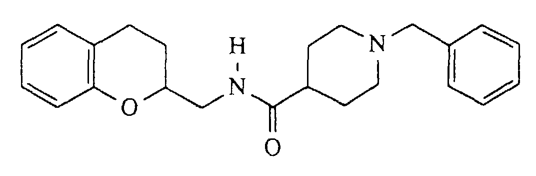

5,0 g (23 mmol) N-Benzylpiperidin-4-carbonsäure werden in 31 ml trockenem Tetrahydrofuran suspendiert und eine Lösung aus 4,1 g (25,3 mmol) 1,1'-Carbonyldiimidazol und 62 ml trockenem Tetrahydrofuran bei 20-25°C in 30 min zugetropft. Nach beendeter Kohlendioxyd-Entwicklung (ca. 1 h) wird noch zusätzlich 1 h gerührt und eine Lösung aus 4,5 g (27,5 mmol) 2-Aminomethyl-3,4-dihydro-2H-1-benzopyran und 31 ml trockenem Tetrahydrofuran in 30 min zugetropft. Anschließend wird der Ansatz noch 18 h bei Raumtemperatur gerührt. Zur Aufarbeitung wird der Ansatz in eine Mischung aus 1,25 1 5%iger Natriumchloridlösung, 56 ml 1 molarer Salzsäure und 0,6 1 Toluol eingerührt. Nachdem die wäßrige Phase noch 1 mal mit 0,3 1 Toluol extrahiert wurde, werden die vereinigten organischen Phasen mit 300 ml 0,1 molarer Salzsäure extrahiert. Nach Abtrennen der wäßrigen Phase beginnt aus der organischen Phase das Produkt als Hydrochlorid auszukristallisieren und kann abgesaugt und getrocknet werden.
Ausbeute: 7,4 g (77% der Theorie)
Fp.: 161-162°C

Aus wäßriger Lösung kann das Hydrochlorid mit Natronlauge in die Base überführt werden. Nach Extraktion mit Toluol kann die Base nach Einengen der toluolischen Lösung auf ca. 70 ml durch zutropfen von 140 ml Petroleumbenzin zum auskristallisieren gebracht werden.
Ausbeute: 5,7g g (68% der Theorie)
Fp.: 122-123°C
DC: Essigester - Ethanol 90:10
R_{f} = 0,28

### Herstellungsbeispiele

### Beispiel 1

S-(+)-2-(N-Cycloheptylmethyl)aminomethyl-chroman Hydrochlorid

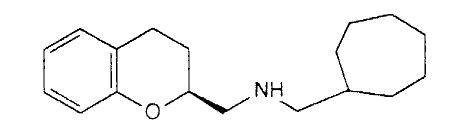

3,2 g (7,7 mmol) (2S)-2-[N-Cycloheptylmethyl-N-((1R)-1-phenethyl)]aminomethyl-chroman Hydrochlorid wird in 40 ml Methanol gelöst und mit 0,5 g 10% Pd auf Aktivkohle verrührt. Nach 15 Minuten wird abfiltriert, mit der gleichen Menge Katalysator sowie 11 ml konzentrierter Salzsäure versetzt und mit 15 ml Methanol verdünnt. Das Gemisch wird 3 Stunden bei Normaldruck und Raumtemperatur hydriert. Der Katalysator wird abfiltriert und das Filtrat eingeengt. Hieraus wird die Base durch Behandeln mit Natriumhydrogencarbonat-Lösung -Lösung und Extraktion mit Essigester gewonnen. Chromatographie auf Kieselgel (Dichlormethan/Ethanol/Triethylamin 400:10:1) ergibt 1,5 g rohe freie Base. Hieraus wird durch Behandeln mit etherischer Salzsäure das Hydrochlorid gewonnen, das aus Acetonitril umkristallisiert wird.
Ausbeute: 1,15 g farblose Kristalle (48%)
Schmp.: 177-178°C (nach Umkrist. aus Acetonitril)
R_{f} = 0,50 (Kieselgel: Dichlormethan/Ethanol/Ammoniak /Ammoniak 200:10:1)
IR (KBr): 3374, 2920 (b), 1582, 1489, 1456
α_{D} (c = 1, Methanol): +94,6°

In Analogie zur Vorschrift des Beispiels 1 werden die in Tabelle 1 aufgeführten Verbindungen hergestellt: (Es handelt sich dabei stets um die Hydrochloride.)

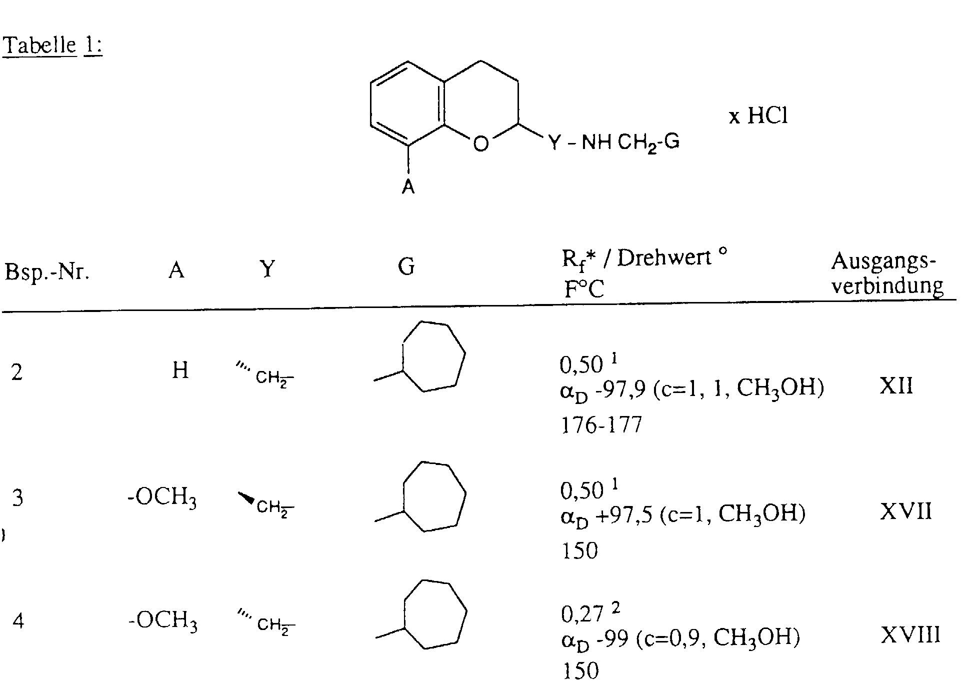

### Beispiel 5

2-[N-(1-Naphthylmethyl)]aminomethyl-8-methoxychroman Hydrochlorid

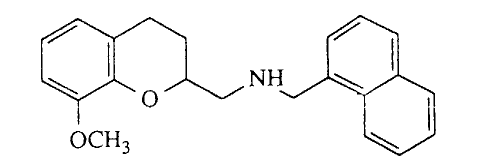

Zu 85 g einer 3,4 M Lösung von Natrium-bis(2-methoxyethoxy)-dihydroaluminat in Toluol, verdünnt mit 100 ml trockenem Toluol werden bei 50°C 32,8 g (95 mmol) N-(1-Naphthylmethyl)-8-methoxy-chroman-2-carbonsäureamid in mehreren Portionen zugegeben. Nach 2 Stunden Rühren bei dieser Temperatur wird mit 150 ml Toluol verdünnt und mit einem Gemisch aus 20 ml Tetrahydrofuran und 8 ml Wasser tropfenweise hydrolysiert. Das Reaktionsgemisch wird über ca. 50 g Kieselgel K₆₀ filtriert. Das Filtrat wird eingeengt und das Rohprodukt durch Flashchromatographie (Kieselgel, Toluol/Essigester 100:0 bis 2:1) gereinigt. Dies ergibt 31,8 g (quantitativ) der Titelverbindung als freie Base. Aus 1,0 g dieser Base wird durch Behandeln mit etherischer Salzsäure das Hydrochlorid gewonnen, das aus 2-Propanol/Diethylether umkristallisiert wird.
Ausbeute: 0,84 g farblose Kristalle.
Schmp.: 145-147°C (nach Umkrist. aus 2-Propanol/Diethylether)
R_{f} = 0,10 (Kieselgel, Toluol/Essigester 1:1)

In Analogie zur Vorschrift des Beispiels 5 werden die in Tabelle 2 aufgeführten Verbindungen hergestellt: (Es handelt sich dabei stets um die Hydrochloride.)

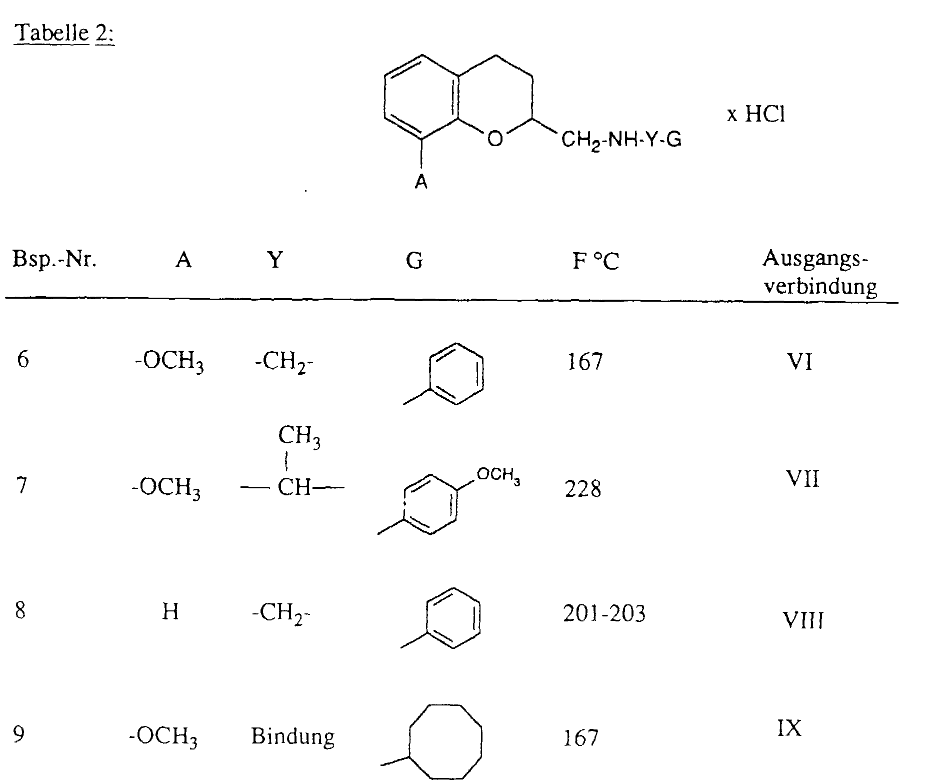

### Beispiel 10

### 2-(N-Cycloheptylmethyl)aminomethyl-chroman Hydrochlorid

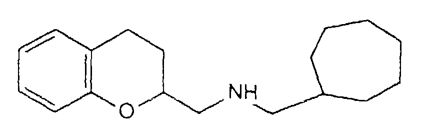

25 mmol der Verbindung aus Beispiel XII werden zu einer Mischung von 20 g (73 mmol) einer 70%igen Lösung von Natrium-bis-(2-methoxyethoxy)-dihydroaluminat in Toluol und 30 ml Toluol unter Argon portionsweise zugegeben. Nach 1 Stunde bei 50°C wird im Eisbad abgekühlt und mit 30 ml Toluol verdünnt. Eis wird in kleinen Portionen zugegeben, bis die Gasentwicklung beendet ist, gefolgt von der Zugabe von ca. 5 ml 45%iger Natronlauge. Flashchromatographie (Kieselgel, Essigester) des Reaktionsgemisches liefert die freie Base als Sirup. Hieraus wird durch Behandeln mit etherischer Salzsäure 5,2 g (67% bezogen auf eingesetztes 2-Aminomethyl-chroman) Hydrochlorid gewonnen.
Schmp.; 194-195°C (nach Umkrist. aus Acetonitril)
IR (KBr): 2929, 2854, 2743(b), 1594

In Analogie zur Vorschrift des Beispiels 10 wird die in Tabelle 3 aufgeführte Verbindung hergestellt: (Die Verbindung liegt als Hydrochlorid vor.)

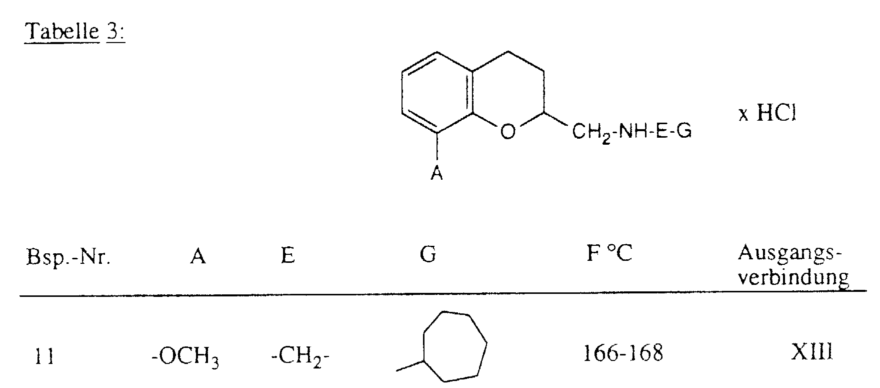

### Beispiel 12

2-(R)-2-(N-Cycloheptylmethyl)aminomethyl-8-hydroxy-chroman Hydrochlorid

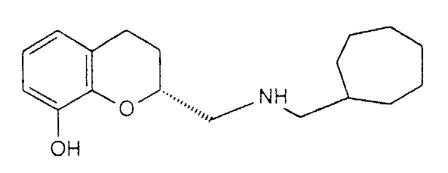

1,7 g (5,0 mmol) 2-(R)-2-(N-Cycloheptylmethyl)aminomethyl-8-methoxy-chroman Hydrochlorid werden mit 50 ml 48% Bromwasserstoffsäure 4,5 Stunden zum Rückfluß erhitzt. Das nach Abkühlen auf Raumtemperatur ausfallende Produkt wird abgesaugt und mit Wasser gewaschen. Durch Behandeln mit Natriunihydrogencarbonat-Lösung / Essigester wird die Base freigesetzt. Die organische Phase wird getrocknet (Magnesiumsulfat) und eingeengt. Hieraus wird durch Umsetzen mit etherischer Salzsäure das Hydrochlorid gewonnen. Umkristallisation aus 2-Propanol liefert 0,58 g (36%) farblose Kristalle.
Schmp.: 250-251°C (nach Umkrist. aus 2-Propanol)
R_{f} = 0,30 (Kieselgel; Dichlormethan/Ethanol/Ammoniak /Ammoniak 200:10:1)
IR (KBr): 3252(b), 2924, 2855, 1595, 1484
α_{D} (c=1, Methanol): -109,7°

### Beispiel 13

2-(R)-2-(N-Cycloheptylmethyl-N-methyl)aminomethyl-8-methoxy-chroman Hydrochlorid

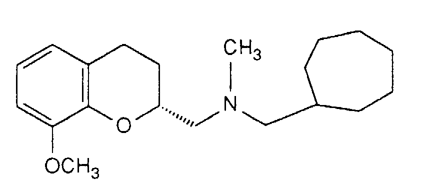

Aus 1,7 g (5 mmol) 2-(R)-2-(N-Cycloheptylmethyl)aminomethyl-8-methoxy-chroman Hydrochlorid wird die Base freigesetzt und in 25 ml Dioxan gelöst. Es werden 25 ml einer wäßrigen 1 M Lösung von NaH₂PO₃ sowie 2,5 ml einer 35% wäßrigen Formaldehyd-Lösung zugesetzt. Nach 15 Minuten bei 60°C wird abgekühlt und zu einer Mischung aus je 100 ml gesättigter Natriumhydrogencarbonat-Lösung und Essigester gegossen. Die wäßrige Phase wird mit Essigester extrahiert. Die vereinigten organischen Extrakte werden getrocknet (Magnesiumsulfat) und eingeengt. Chromatographie (Kieselgel, Dichlormethan / Ethanol / Triethylamin 400:10:1) liefert die freie Base, die durch Behandeln mit etherischer Salzsäure in das Hydrochlorid überführt wird. Umkristallisation aus Acetonitril ergibt 0,45 g (28%) farblose Kristalle. Schmp.: 203-204°C (nach Umkrist. aus Acetonitril)
R_{f} = 0,24 (Kieselgel, Dichlormethan/Ethanol/Ammoniak 400:10:1)
IR (KBr): 3422(b), 2924, 2853, 2560(b), 1587, 1486, 1467
α_{D} (c=0,9, Methanol): -92,2°

### Beispiel 14

2-(R)-{4-[4-(4-Phenoxybutoxy)phenyl]butyl}aminomethyl-8-methoxy-chroman Hydrochlorid

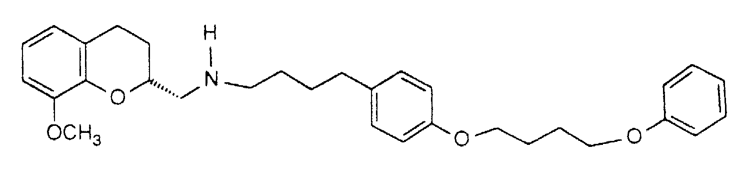

3,95 g (6,7 mmol) 2-(R)-(2-{N-[1-(R)-1-Phenethyl-N-{4-[4-(4-Phenoxybutoxy)phenyl]-butyl}}aminomethyl-8-methoxy-chroman Hydrochlorid in 40 ml Methanol werden mit 1 g 5% Pd/C ausgerührt. Nach Filtration wird dem Filtrat 13,4 ml konzentrierte Salzsäure und 1,0 g 5% Pd auf Aktivkohle (suspendiert in 10 ml Methanol) zugesetzt. Es wird 4 Stunden bei Normaldruck hydriert. Die Reaktionsmischung wird mit 40 ml Tetrahydrofuran und 100 ml Dichlormethan verdünnt und über Kieselgur filtiert. Einengen bis zu beginnenden Kristallisation liefert nach Stehen über Nacht 1,6 g Rohprodukt, das zuerst aus Methanol, dann aus 2-Propanol umkristallisiert wird.
Ausbeute: 0.80 g (23%) farblose Kristalle
Schmp.: 125°C
R_{f} = 0,44 (Kieselgel, Dichlormethan/Methanol 10:1)

### Beispiel 15

2-N-(1,4-Dioxaspiro[4,5]decan-8-yl)aminomethyl-8-methoxychroman Hydrochlorid

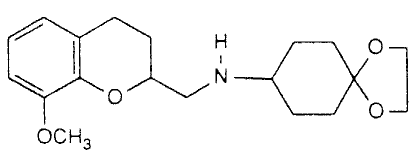

Aus 2,3 g (10 mmol) 2-Aminomethyl-8-methoxychroman Hydrochlorid wird mit Natriumhydrogencarbonat die Base freigesetzt. Diese wird in 30 ml Methanol gelöst und mit 1,2 g (20 mmol) Essigsäure sowie 3,2 g (20 mmol) 1,4-Dioxaspiro[4,5]decan-8-on versetzt. Nach Zugabe von 10 g Molsieb 4 Å wird 30 min bei Raumtemperatur und anschließend 30 min bei 50°C gerührt. Nach Abkühlen auf 0°C werden 1,3 g Natriumcyanoborhydrid zugegeben. Die Reaktionsmischung wird 2 Stunden auf 50°C erhitzt. Nach Abkühlen auf Raumtemperatur wird filtriert und das Filtrat eingeengt. Der Rückstand wird zwischen 1 N Natronlauge und Dichlormethan verteilt. Trocknen der organischen Phase und Einengen liefert ein Rohprodukt, das durch Flashchromatographie (Kieselgel, Toluol / Essigester-Gradient 100:0 bis 0:100, dann Essigester / 2-Propanol / Triethylamin 75:25:1) gereinigt wird. Die freie Base der Titelverbindung wird als Öl erhalten, das durch Behandeln mit etherischer Salzsäure in das Hydrochlorid überführt wird.
Ausbeute: 2,4 g (66%) farblose Kristalle
Schmp.: 236-237°C (nach Umkrist. aus Acetonitril)
IR (KBr): 3510, 2951(b), 2752(b), 1587, 1486

### Beispiel 16

N-Cyclopentylmethyl-2-aminomethylchroman

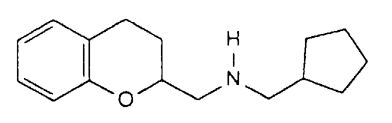

Zu 300 ml einer 1 M Boran/Tetrahydrofuran-Lösung -Lösung (0,3 mol) werden bei 0 bis -10°C unter Rühren und Argon innerhalb von 30 Minuten 16,0 g (0,06 mol) N-Cyclopentylmethyl-chroman-2-carbonsäureamid in 200 ml wasserfreiem Tetrahydrofuran getropft. Der Ansatz wird 2 h bei 20°C und anschließend 6 h bei 60°C gerührt. Nach Stehenlassen über Nacht werden 80 ml 10%ige Salzsäure zugetropft. Nach 3 stündigem Nachrühren bei 20°C wird der Ansatz im Wasserstrahlvakuum zur Trockene eingedampft. Der Rückstand wird mit 10%iger Natronlauge alkalische gestellt. Extrahieren mit Dichlormethan, Waschen der vereinigten Dichlormethanextrakte mit gesättigter Kochsalzlösung und Eindampfen des über wasserfreiem Natriumsulfat getrockneten Extraktes ergeben 16,0 g rohes Amin. Destillation liefert 11,5 g der Titelverbindung vom Sdp. 120-126°C/0,2 / 0,2 - 0,25 Torr.

### Beispiel 17

N-Cyclohexylmethyl-2-aminomethyl-8-methoxychroman Hydrochlorid

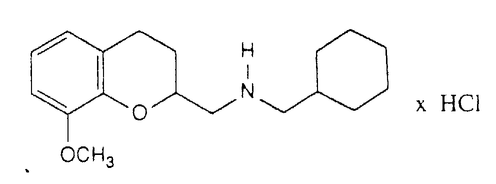

7,2 (15 mmol) N-Cyclohexylmethyl-2-aminomethyl-8-methoxychroman werden in 200 ml Diethylether unter Rühren bei 0 bis -10°C mit 9,3 ml einer 2,7 M Chlorwasserstofflösung in Diethylether versetzt. Nach 2 h wird der Niederschlag abfiltriert. gründlich mit Diethylether gewaschen und bei 60°C / 0,01 mbar getrocknet.
Ausbeute: 6,6 h (81%)
Schmp.: 188-191°C

In Analogie zu den oben aufgeführten Vorschriften werden die in den Tabellen 4, 5, 6, 7, 8, 9 und 10 aufgeführten Verbindungen hergestellt:

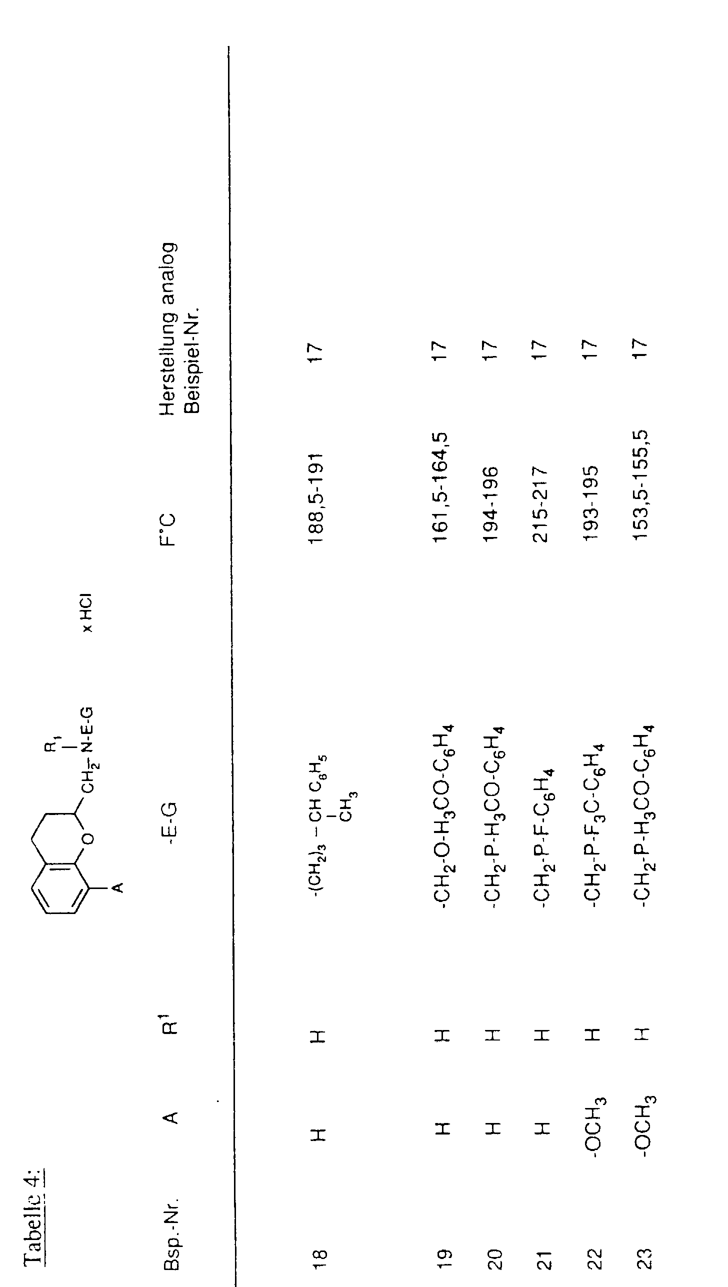

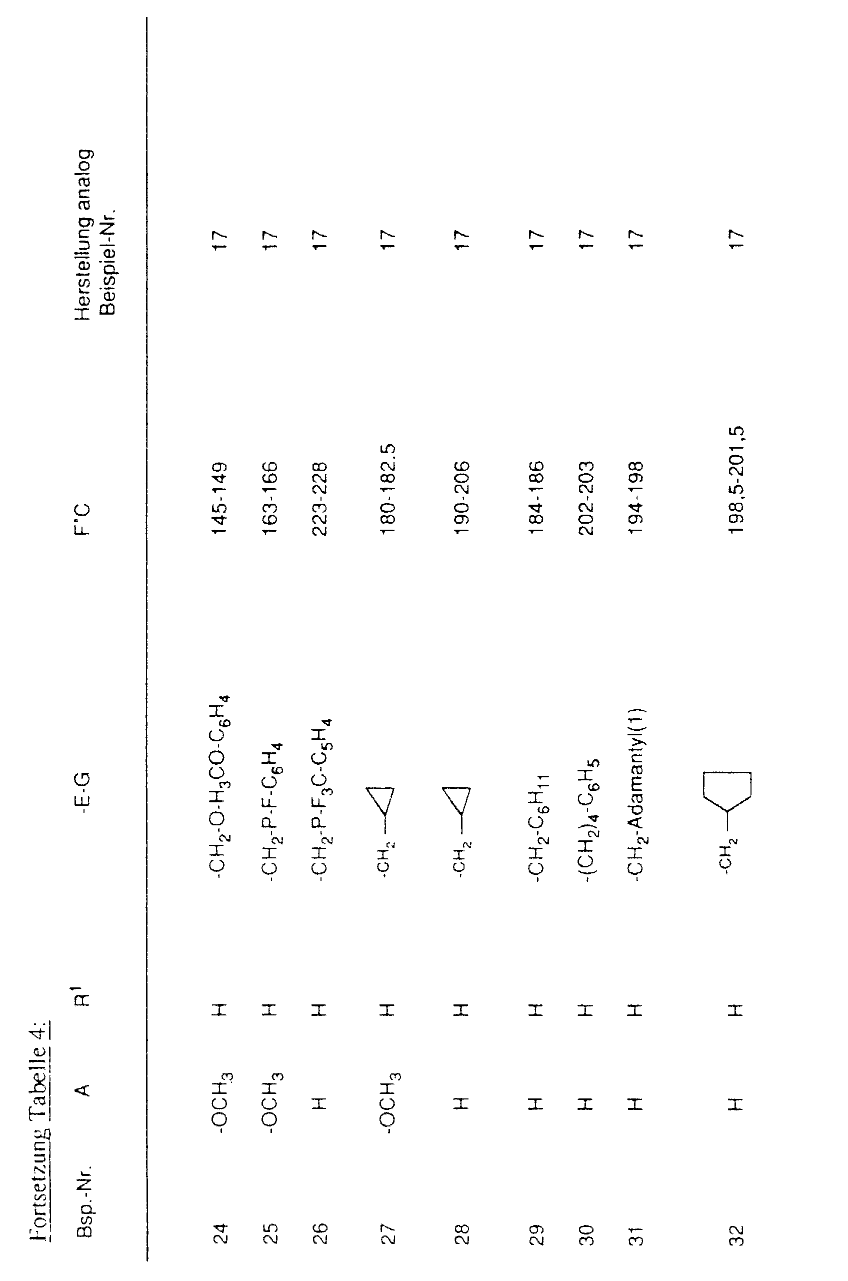

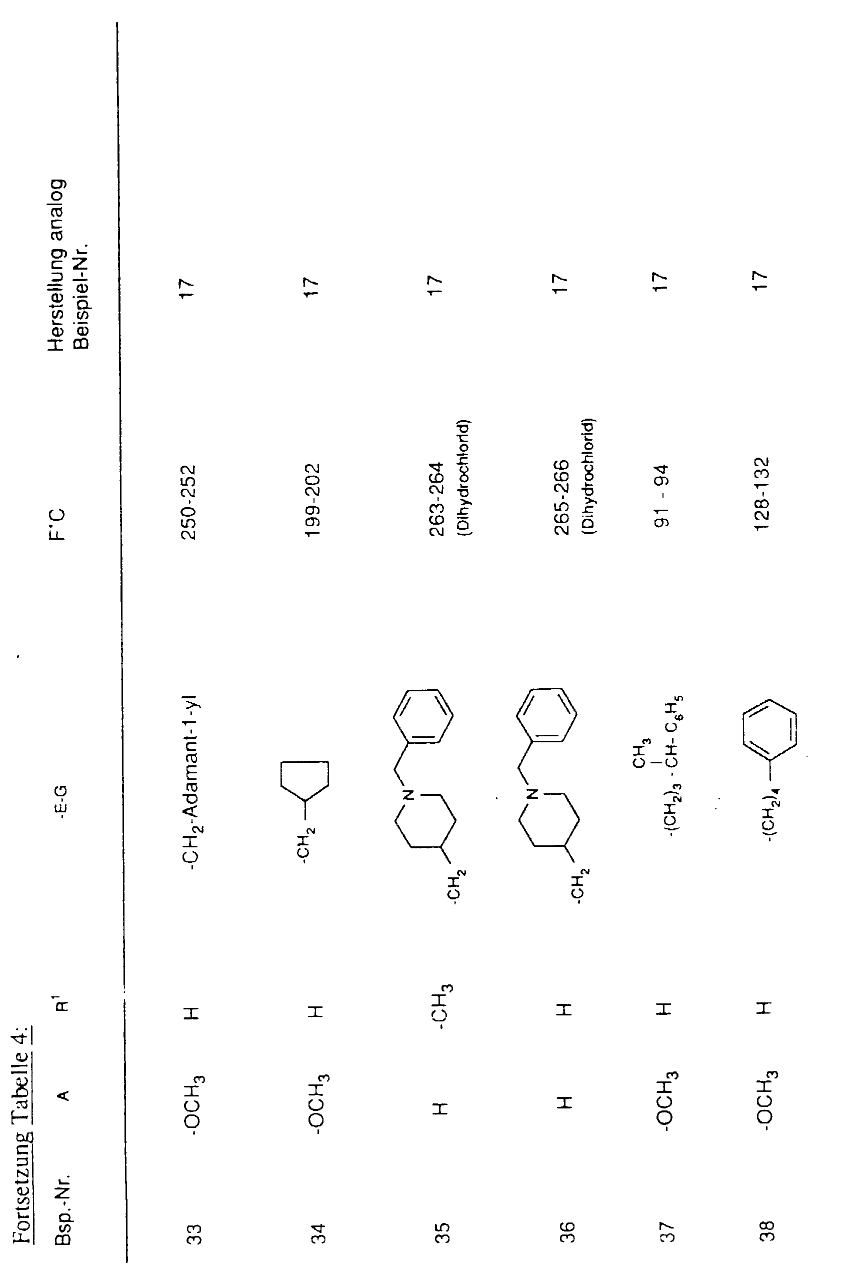

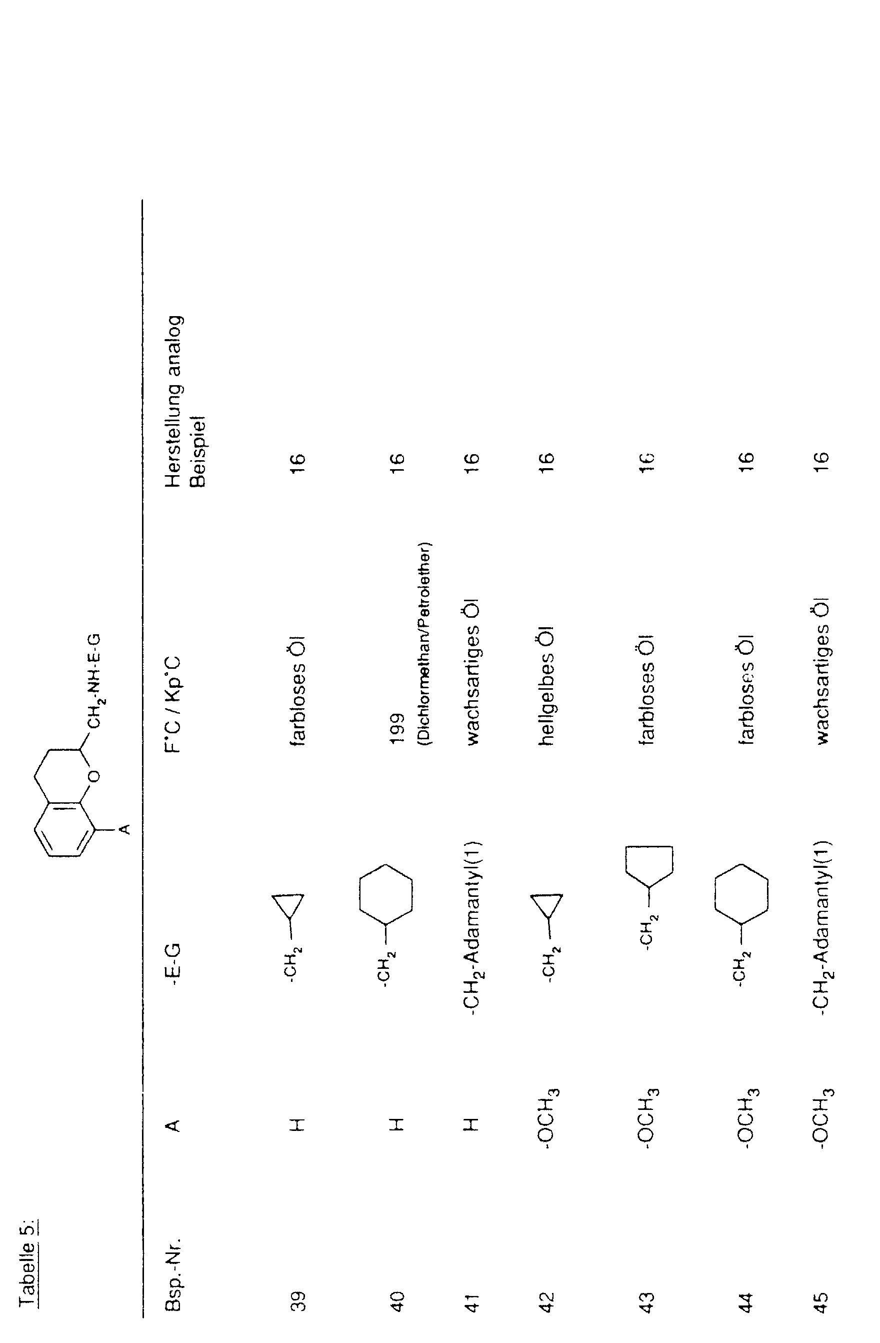

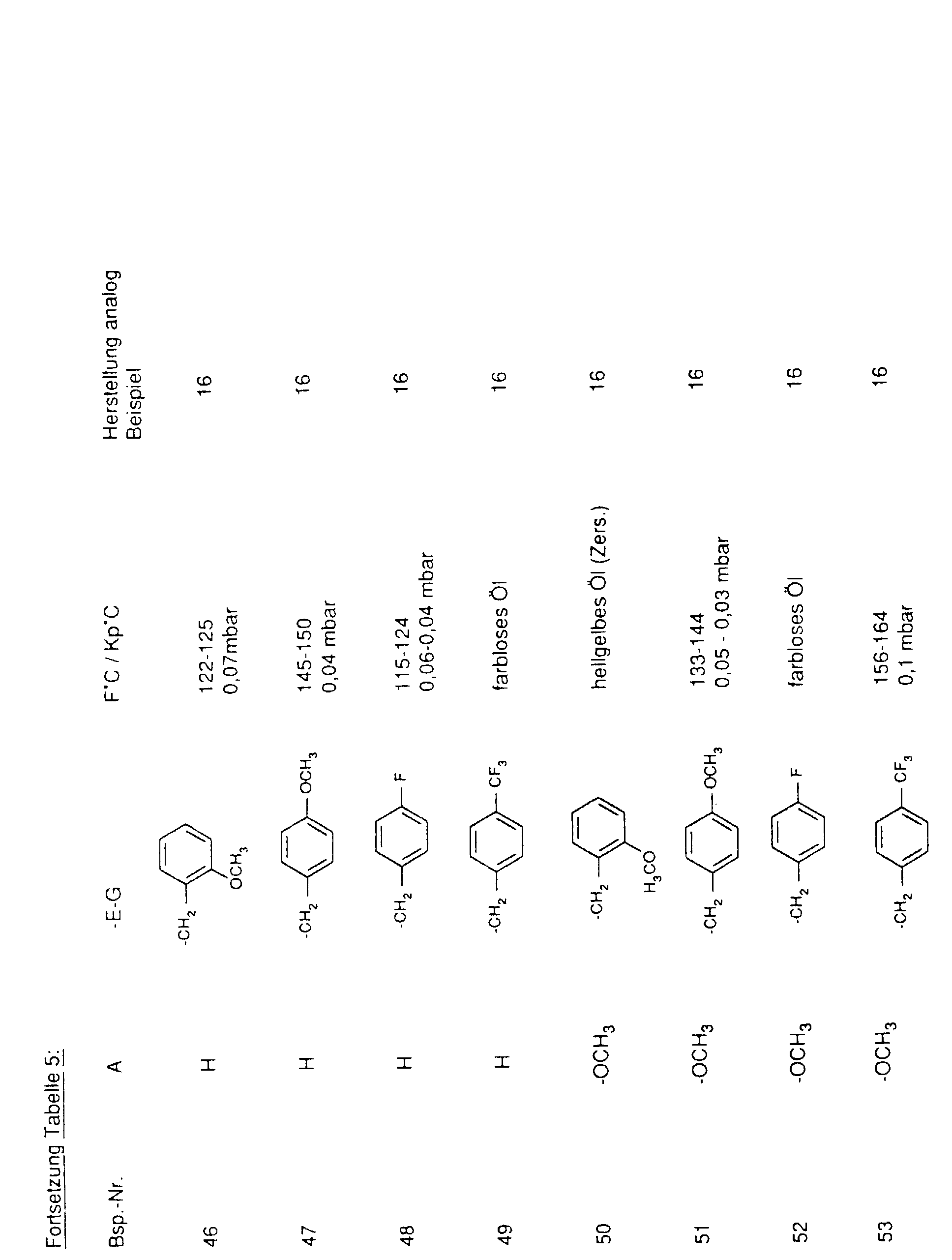

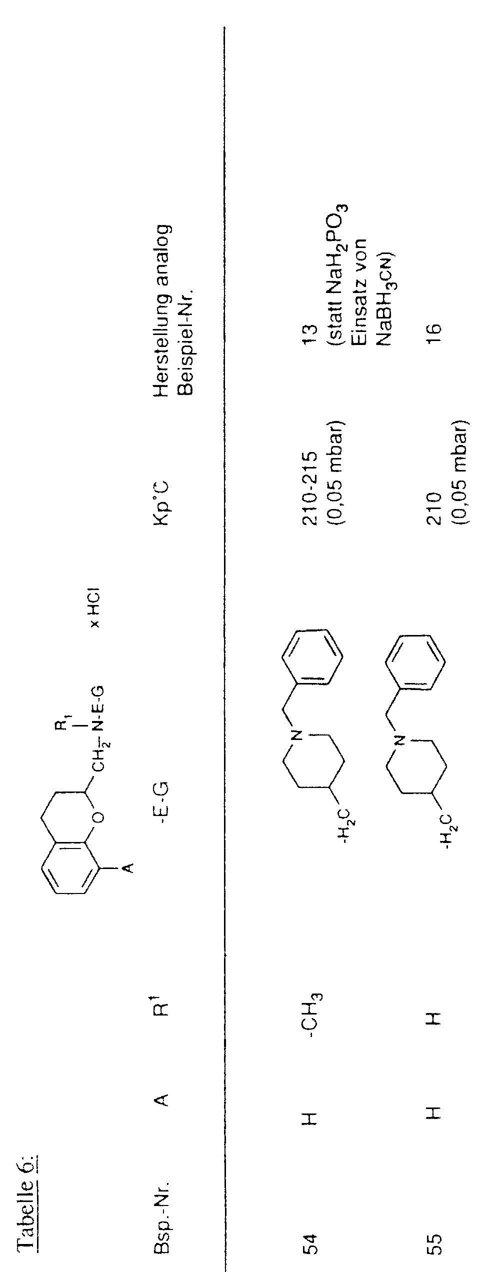

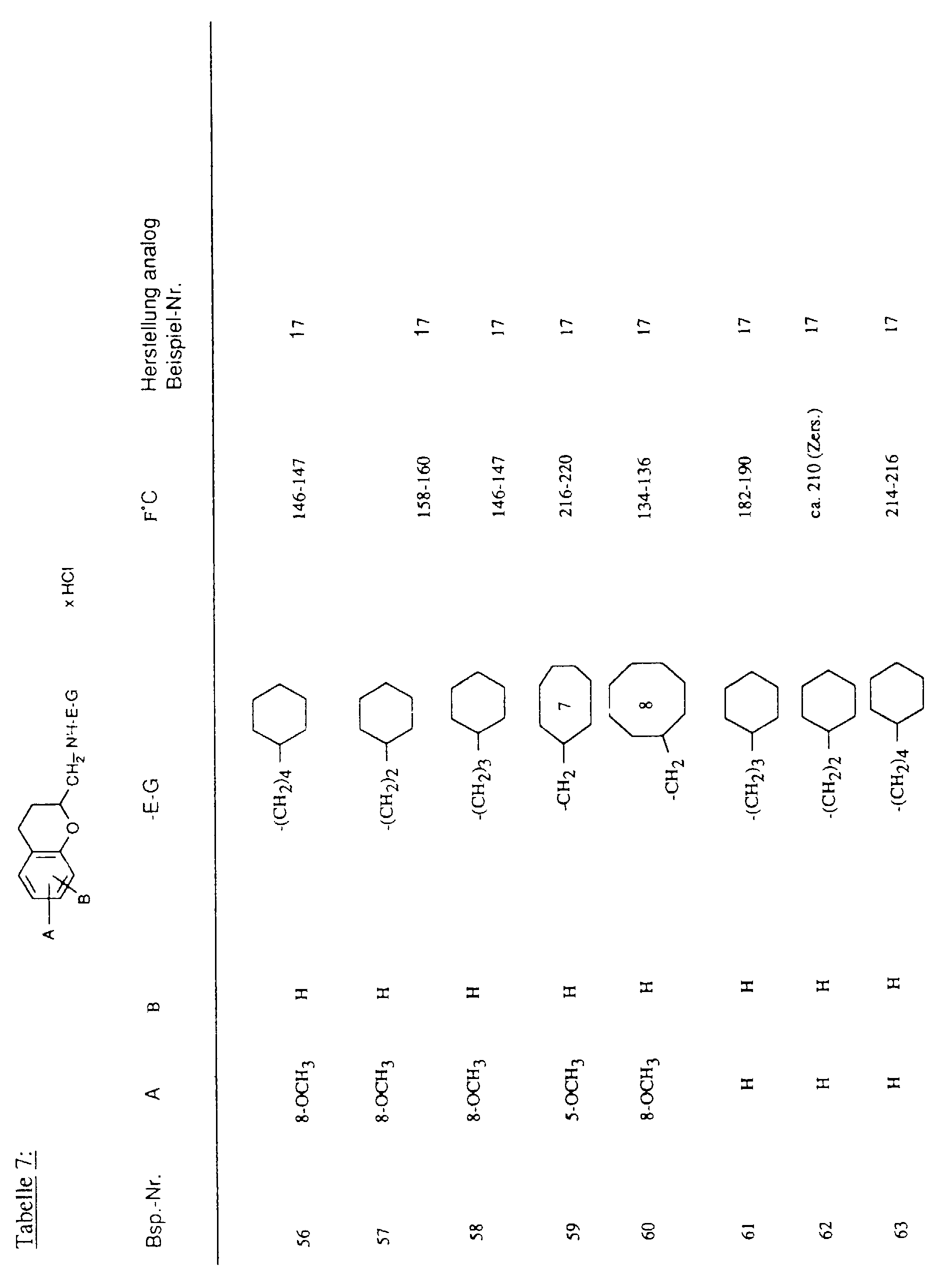

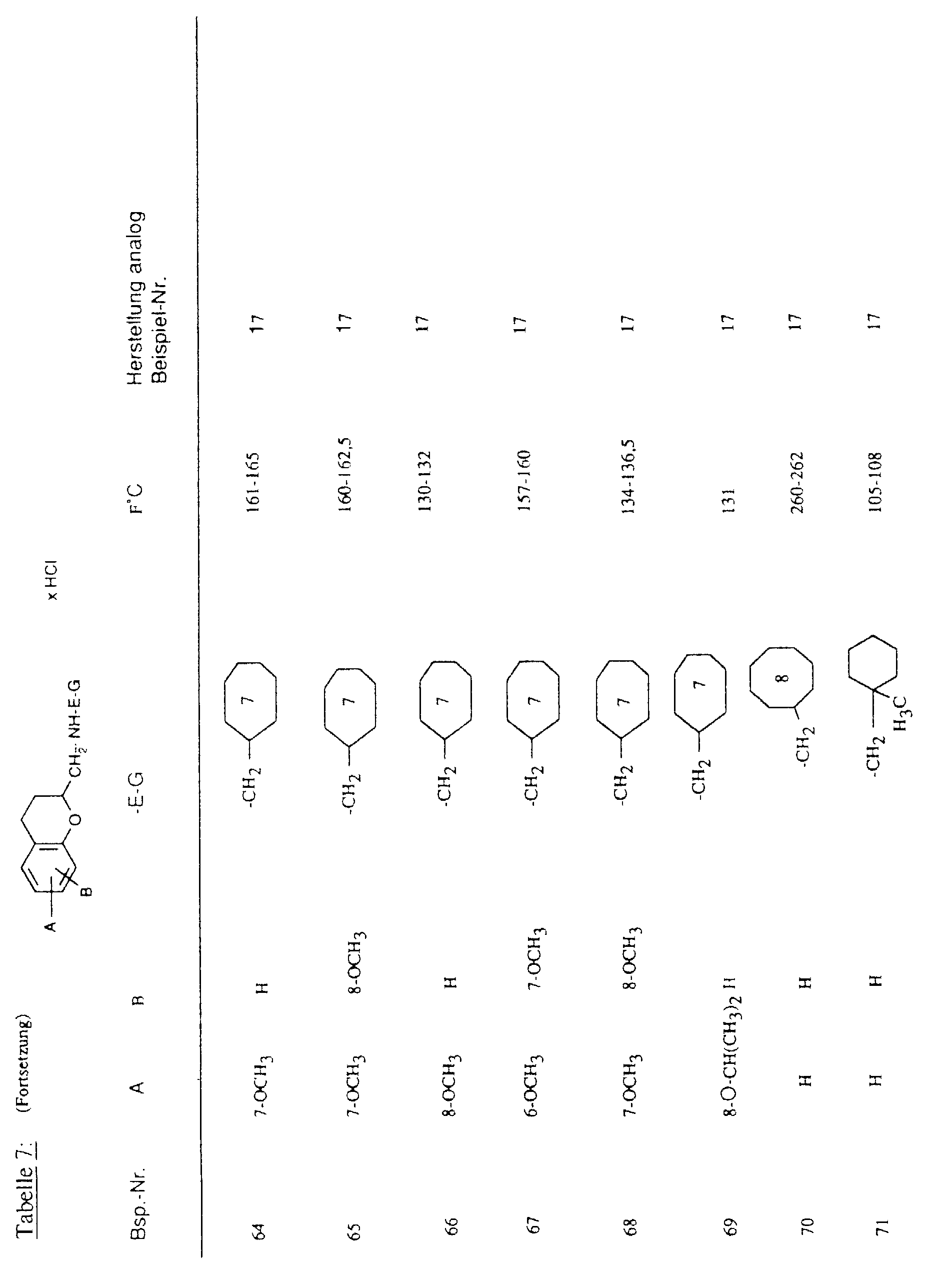

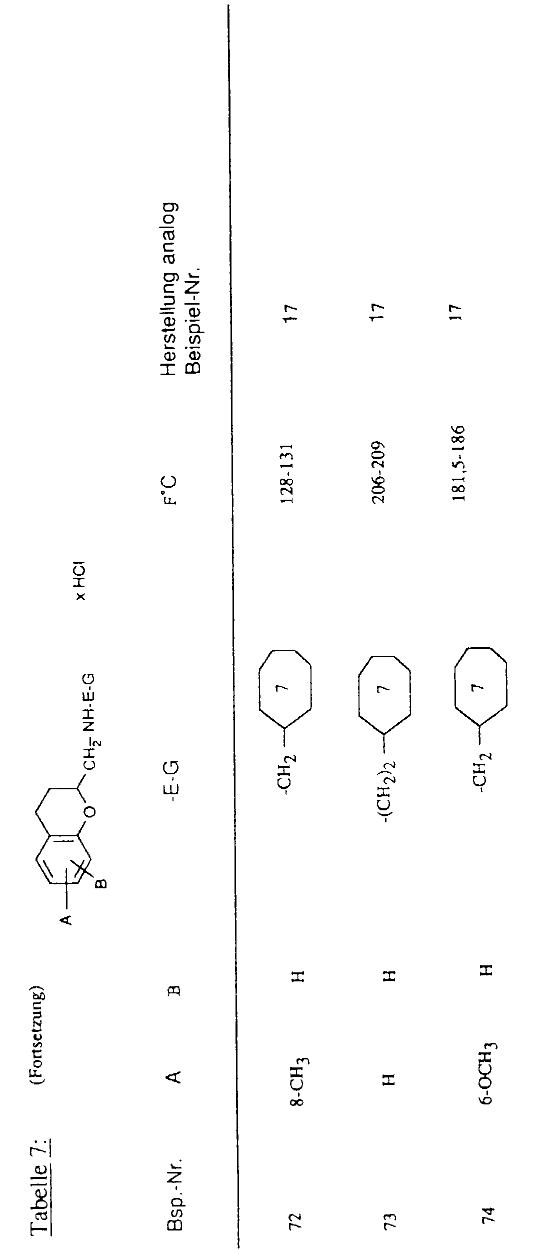

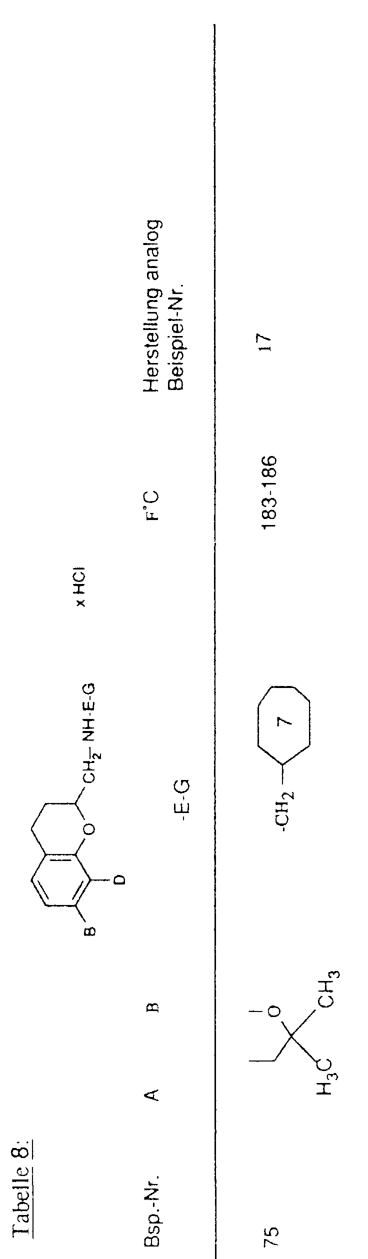

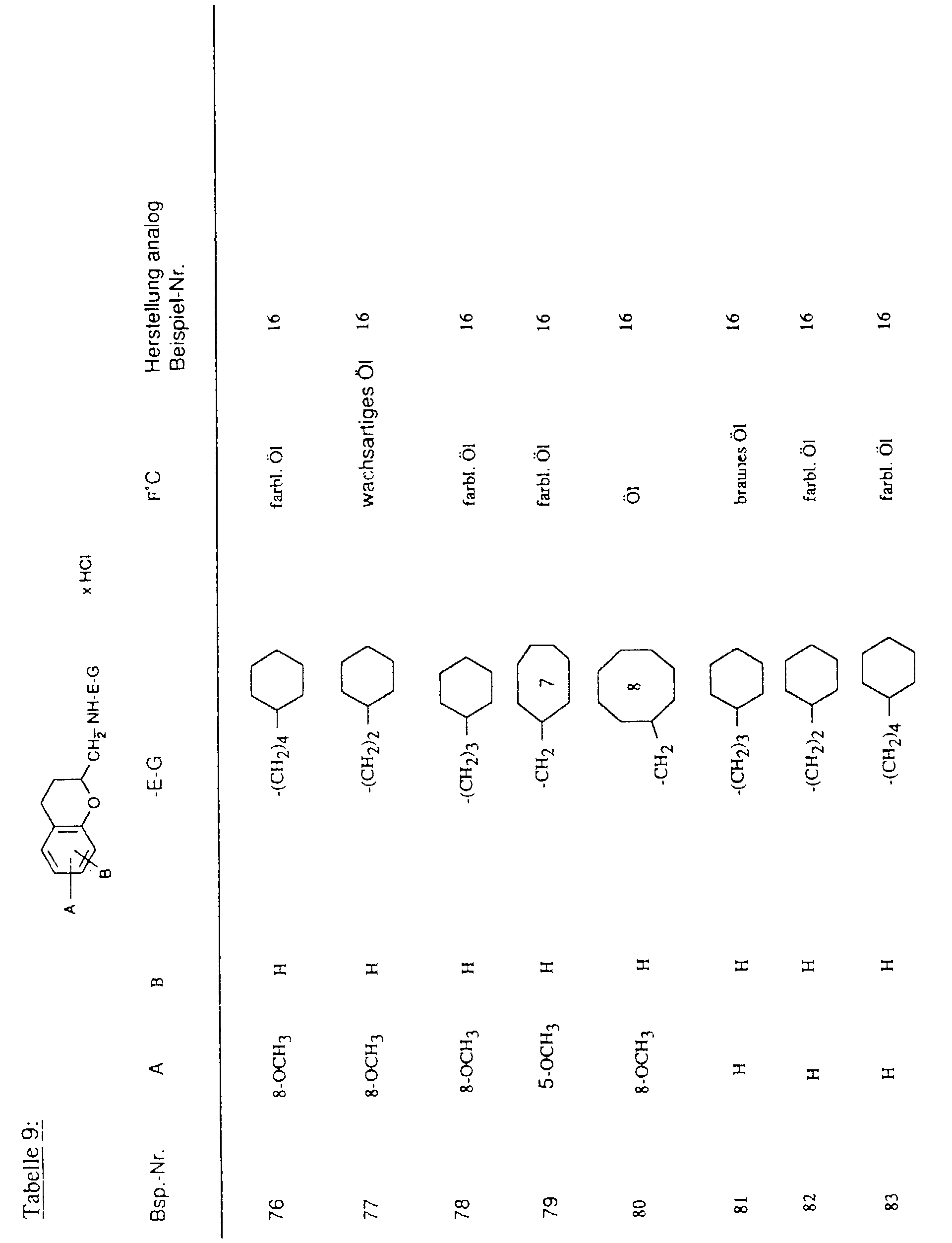

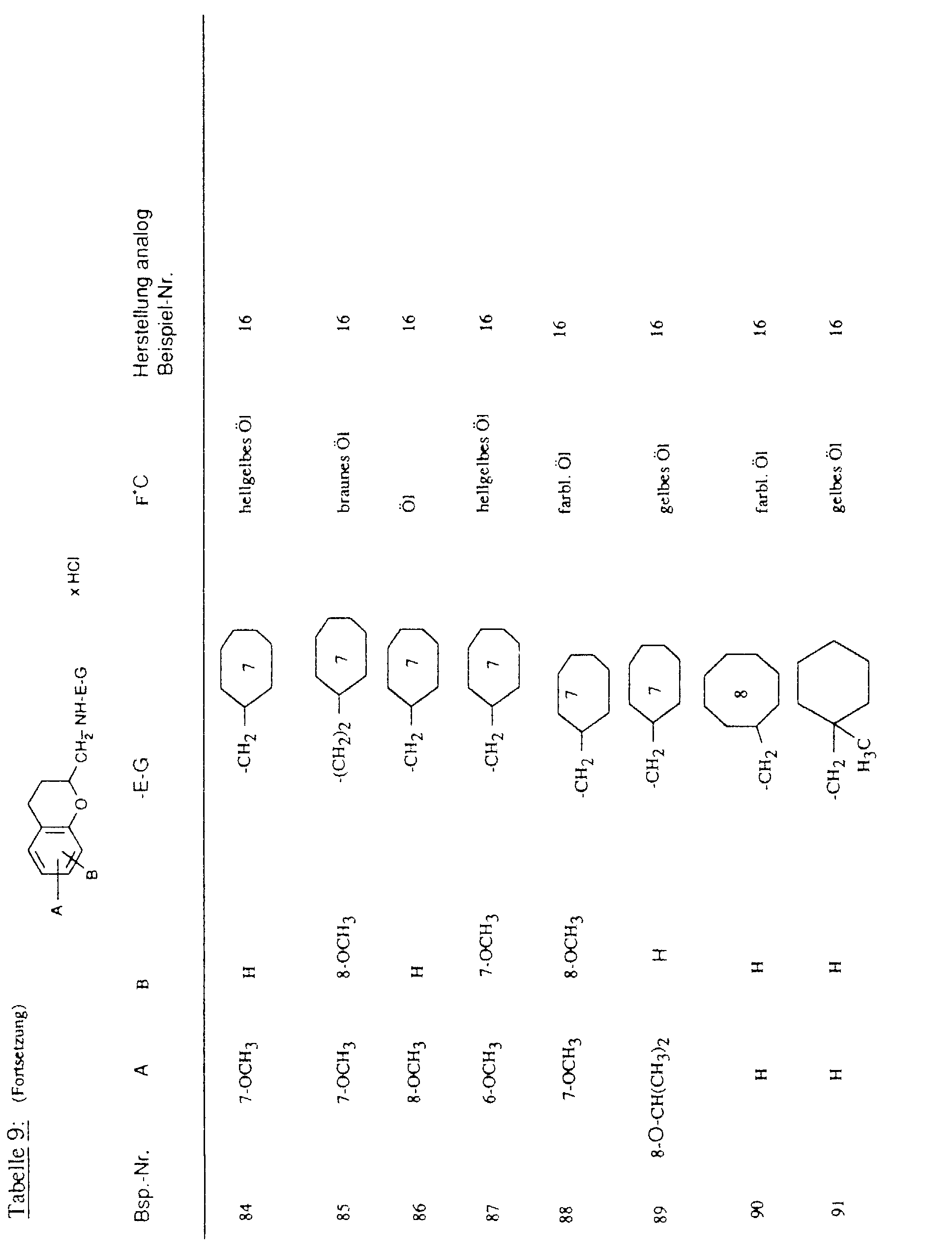

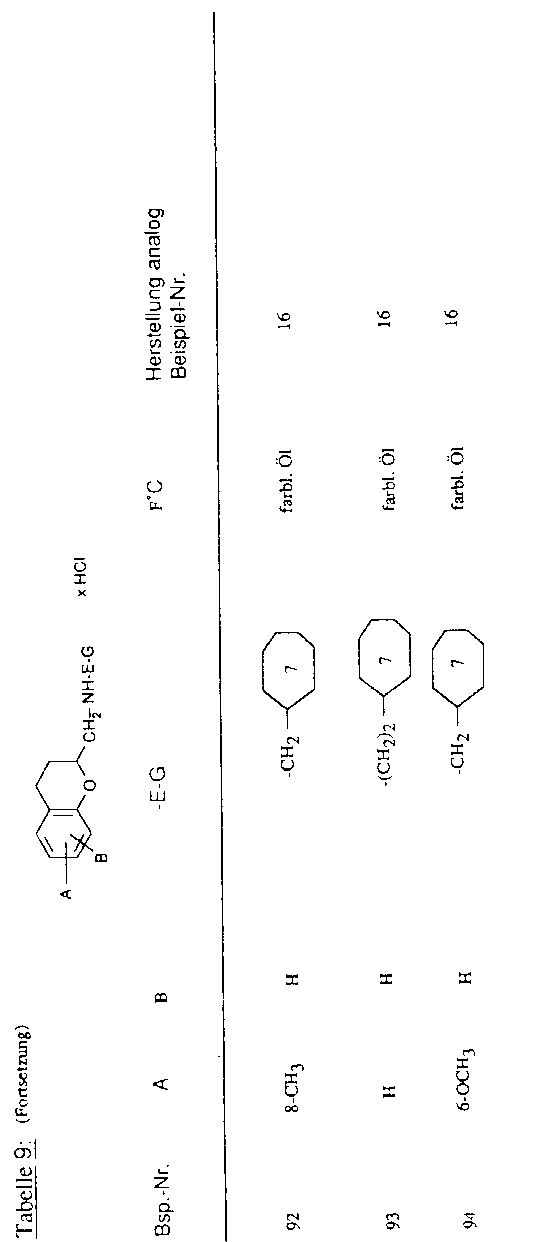

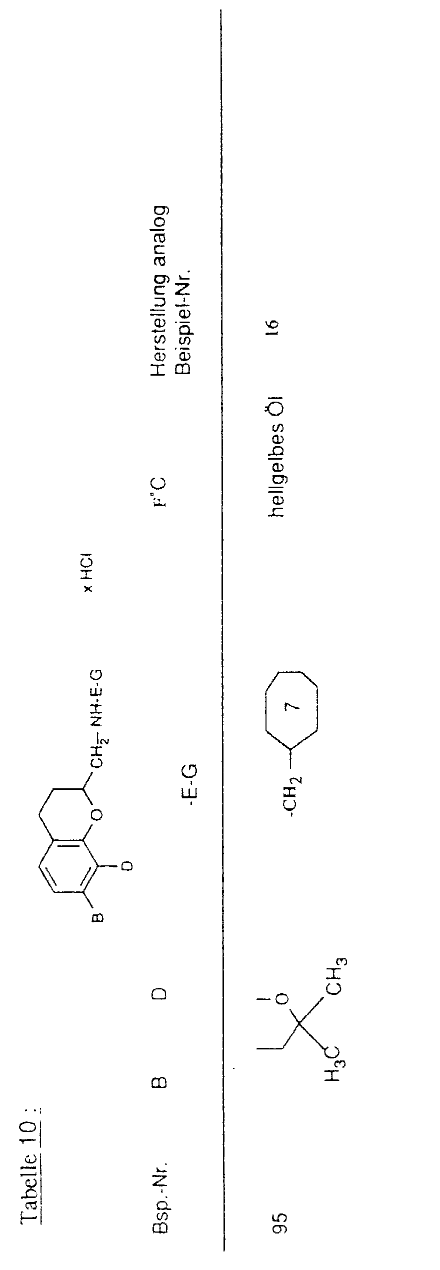

## Patentansprüche

1. Aminomethyl-chromane der allgemeinen Formel (I) 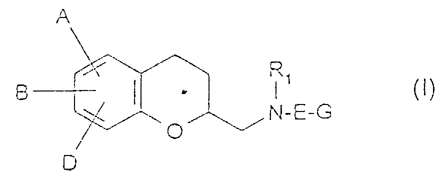 in welcher
A, B und D gleich oder verschieden sind und
für Wasserstoff, Fluor, Chlor, Brom, Cyano, Trifluormethyl, Difluormethoxy, Trifluormethoxy oder Hydroxy stehen,
für geradkettiges oder verzweigtes Alkyl, Alkenyl, Acyl oder Alkoxycarbonyl mit jeweils bis zu 6 Kohlenstoffatomen stehen,
für eine Gruppe der Formel -NR²R³, -NR⁴-L-R⁵ oder -OR⁶ stehen,
R², R³ und R⁴ gleich oder verschieden sind und Wasserstoff oder geradkettiges oder verzweigtes Alkyl mit bis zu 6 Kohlenstoffatomen bedeuten,
L die -CO- oder SO₂-Gruppe bedeutet
R⁵ geradkettiges oder verzweigtes Alkyl mit bis zu 6 Kohlenstoffatomen oder Benzyl bedeutet, oder
Phenyl bedeutet, das gegebenenfalls durch Fluor, Chlor, Brom, Trifluormethyl, Trifluormethoxy, Hydroxy oder durch geradkettiges oder verzweigtes Alkyl oder Alkoxy mit jeweils bis zu 4 Kohlenstoffatomen substituiert ist,
R⁶ geradkettiges oder verzweigtes Alkyl oder Alkenyl mit bis zu 6 Kohlenstoffatomen bedeutet, die gegebenenfalls durch Cyclopropyl, Cyclopentyl, Cyclohexyl oder Phenyl substituiert sind,
oder
A eine der oben aufgeführten Bedeutungen hat
und
B und D gemeinsam einen Rest der Formel 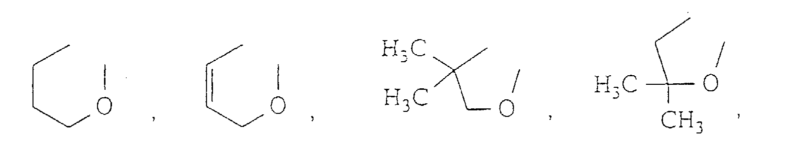 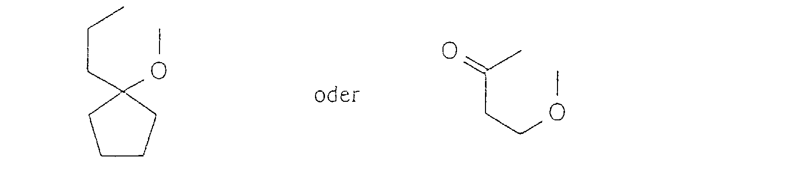 bilden,
E für eine direkte Bindung steht, oder
für geradkettiges oder verzweigtes Alkylen, Alkenylen oder Alkinylen mit jeweils bis zu 8 Kohlenstoffatomen steht, die gegebenenfalls durch Phenyl substituiert sind,
G für Phenyl, Naphthyl, Pyridyl, Chinolyl, Cyclopropyl, Cyclobutyl, Cyclopentyl, Cyclohexyl, Cycloheptyl, Cyclooctyl oder Adamantyl steht, die gegebenenfalls bis zu 2-fach gleich oder verschieden durch Hydroxy, Fluor, Chlor, Brom, Nitro, Cyano Difluormethyl, Trifluormethyl, Difluormethoxy, Trifluormethoxy oder durch geradkettiges oder verzweigtes Alkyl oder Alkoxy mit jeweils bis zu 6 Kohlenstoffatomen substituiert sind, wobei letztere gegebenenfalls durch Phenyl oder Phenoxy substituiert sind, oder
für einen Rest der Formel 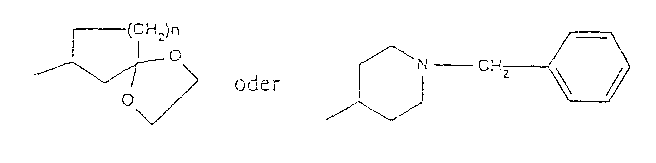 worin
n eine Zahl 1 oder 2 bedeutet,
R¹ für Wasserstoff,
für den Rest der Formel -E'-G' steht,
worin
E' und G'
die oben für E und G angegebene Bedeutung haben und mit diesem gleich oder verschieden sind,
gegebenenfalls in einer isomeren Form,
und deren Salze,
wobei N-[1-Phenylethyl]-2-aminomethyl-chroman und
N-[1-phenylethyl]-2-aminomethyl-8-methoxy-chroman ausgenommen sind.

2. Aminomethyl-chromane nach Anspruch 1, wobei
A, B und D gleich oder verschieden sind und
für Wasserstoff, Fluor, Chlor, Brom, Cyano, Trifluormethyl, Trifluormethoxy oder Hydroxy stehen,
für geradkettiges oder verzweigtes Alkyl oder Alkenyl mit jeweils bis zu 4 Kohlenstoffatomen stehen,
für eine Gruppe der Formel -NR²R³ oder -OR⁶ stehen,
worin
R² und R³ gleich oder verschieden sind und Wasserstoff oder geradkettiges oder verzweigtes Alkyl mit bis zu 4 Kohlenstoffatomen bedeuten,
R⁶ geradkettiges oder verzweigtes Alkyl oder Alkenyl mit bis zu 4 Kohlenstoffatomen bedeutet, die gegebenenfalls durch Cyclopropyl oder Phenyl substituiert sind,
oder
A eine der oben aufgeführten Bedeutungen hat
und
B und D gemeinsam einen Rest der Formel 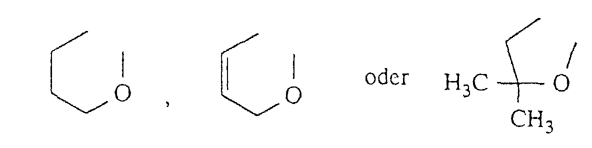 bilden,
E für eine direkte Bindung steht, oder
für geradkettiges oder verzweigtes Alkylen oder Alkenylen mit jeweils bis zu 7 Kohlenstoffatomen steht, die gegebenenfalls durch Phenyl substituiert sind,
G für Phenyl, Naphthyl, Adamantyl, Cyclopropyl, Cyclopentyl, Cyclohexyl, Cycloheptyl oder Cyclooctyl steht, die gegebenenfalls durch Fluor, Chlor, Brom, Hydroxy, Difluormethyl, Trifluormethyl, Difluormethoxy, Trifluormethoxy oder durch geradkettiges oder verzweigtes Alkyl oder Alkoxy mit jeweils bis zu 4 Kohlenstoffatomen substituiert sind, wobei letztere gegebenenfalls durch Phenyl oder Phenoxy substituiert sind, oder
für einen Rest der Formel 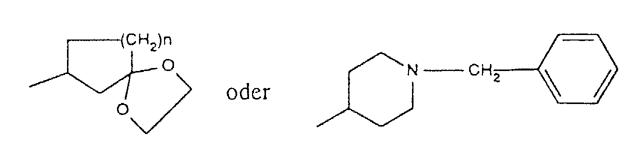 steht,
worin
n eine Zahl 1 oder 2 bedeutet,
R¹ für Wasserstoff oder für den Rest der Formel -E'-G' steht,
worin
E' und G' die obe für E und G angegebene Bedeutung haben und mit diesen gleich oder verschieden sind,
gegebenenfalls in einer isomeren Form,
und deren Salze
wobei N-[1-Phenylethyl]-2-aminomethyl-chroman und N-[1-phenylethyl]-2-aminomethyl-8-methoxy-chroman ausgenommen sind.

3. Aminomethyl-chromane nach Anspruch 1 zur Bekämpfung von Krankheiten.

4. Verfahren zur Herstellung von Aminomethyl-chromanen nach Anspruch 1 und 2 dadurch gekennzeichnet, daß man
[A1] Verbindungen der allgemeinen Formel (II) 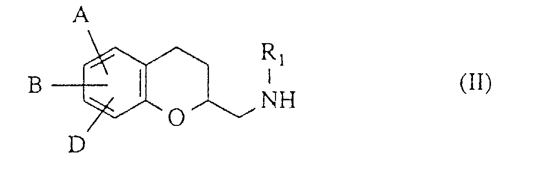 in welcher
A, B, D und R¹ die oben angegebene Bedeutung haben,
mit Verbindungen der allgemeinen Formel (III)
M-E-G (III)
in welcher
M für eine typische Abgangsgruppe wie Chlor, Brom, Iod, Tosylat, Mesylat oder für die Gruppe -OSO₂CF₃ steht,
und
E und G die oben angegebene Bedeutung haben,
in inerten Lösemitteln, gegebenenfalls in Anwesenheit einer Base und/oder eines Katalysators alkyliert,
oder
[A2] Verbindungen der allgemeinen Formel (IV) 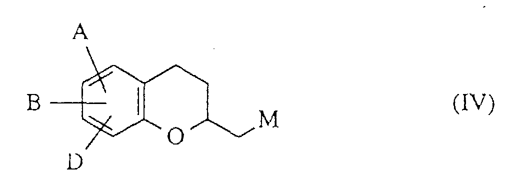 in welcher
A, B, D und M die oben angegebene Bedeutung haben,
mit Aminen der allgemeinen Formel (V) 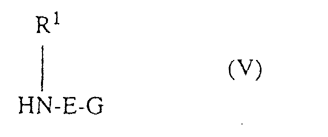 in welcher
R¹, E und G die oben angegebene Bedeutung haben,
in inerten Lösemitteln, gegebenenfalls in Anwesenheit einer Base und/oder eines Katalysators alkyliert,
oder
[B1] Aldehyde der allgemeinen Formel (VI) 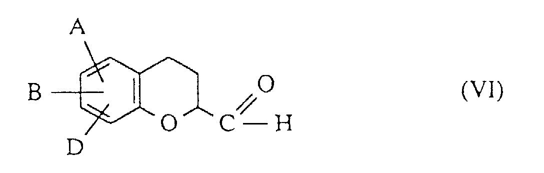 in welcher
A, B und D die oben angegebene Bedeutung haben,
mit Aminen der allgemeinen Formel (V)
in inerten Lösemitteln, gegebenenfalls in Anwesenheit von Hilfsstoffen reduktiv alkyliert,
oder
[B2] im Fall, daß E keine direkte Bindung bedeutet,
Verbindungen der allgemeinen Formel (II) entweder mit Aldehyden der allgemeinen Formel (VII) 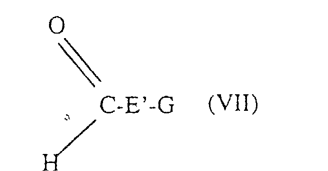 in welcher
G die oben angegebene Bedeutung hat,
E' die oben angegebene Bedeutung von E hat, aber um eine -CH₂-Gruppe verkürzt ist,
oder im Fall, daß E für eine direkte Bindung steht, mit Verbindungen der allgemeinen Formel (VIIa)
O=G' (VIIa)
in welcher
G' die oben angegebene Bedeutung von G hat, aber nicht für Aryl steht,
in inerten Lösemitteln, gegebenenfalls in Anwesenheit von Hilfsstoffen reduktiv alkyliert
oder
[C] Verbindungen der allgemeinen Fonneln (VIII) oder (IX) 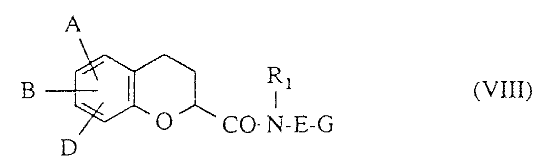 oder 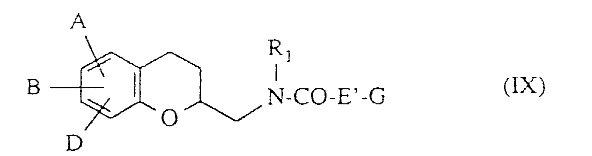 in welcher
A, B, D, E, E', G und R¹ die oben angegebene Bedeutung haben,
in inerten Lösemitteln, gegebenenfalls in Anwesenheit von Hilfsstoffen, nach üblicher Methode reduziert,
oder
[D] im Fall, daß E für 3 bis 6 Methylengruppen steht,
Verbindungen der allgemeinen Formel (IIa) 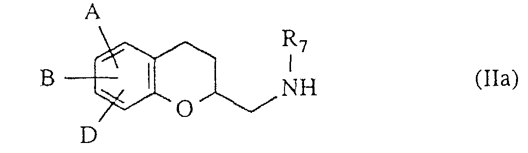 in welcher
A, B und D die oben angegebene Bedeutung haben,
und
R⁷ die oben angegebene Bedeutung von R¹ hat, aber nicht für Wasserstoff steht,
zunächst entweder mit Formaldehyd oder Formaldehydderivaten und mit Verbindungen der allgemeinen Formel (X)
E"-G (X)
in welcher
G die oben angegebene Bedeutung hat
und
E" für einen Rest der Formel HC≡C-(CH₂)_{z} steht,
worin
z eine Zahl 0, 1, 2 oder 3 bedeutet,
in einer Mannichanalogen Reaktion umsetzt und gegebenenfalls in einem nächsten Schritt eine Hydrierung nach üblicher Methode anschließt,
und im Fall, daß R¹ nicht für Wasserstoff steht, entweder nach üblicher Methode alkyliert oder mit Formaldehyd (R¹ = CH₃), wie oben beschrieben, reduktiv alkyliert,
und im Fall, daß R¹ Wasserstoff bedeutet, gegebenenfalls während einzelner Verfahrensschritte zunächst die Aminfunktion mit geeigneten Aminoschutzgruppen blockliert und diese nach üblicher Methode, vorzugsweise durch Hydrogenolyse abspalten,
und die Substituenten A, B, D und G nach üblicher Methode gegebenenfalls modifiziert.

5. Arzneimittel enthaltend mindestens ein Aminomethyl-croman nach Anspruch 1 und 2.

6. Arzneimittel nach Anspruch 5 zur Behandlung von Erkrankungen des Zentralnervensystems.

7. Arzneimittel mach Anspruch 5 zur Behandlung von Krankheiten, die durch Störungen des serotoninergen Systems gekennzeichnet sind.

8. Verwendung von Aminomethyl-chromanen nach Anspruch 1 und 2 zur Herstellung von Arzneimitteln.

9. Verfahren zur Herstellung von Arzneimitteln nach Anspruch 5, dadurch gekennzeichnet, daß man die Aminomethyl-chromane gegebenenfalls mit üblichen Hilfs- und Trägerstoffen in eine geeignete Applikationsform überführt.

## Claims

1. Aminomethyl-chromans of the general formula (I) 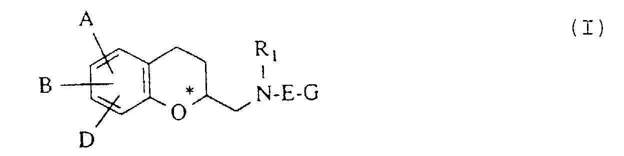 in which
A, B and D are identical or different and
represent hydrogen, fluorine, chlorine, bromine, cyano, trifluoromethyl, difluoromethoxy, trifluoromethoxy or hydroxyl, or
represent straight-chain or branched alkyl, alkenyl, acyl or alkoxycarbonyl having in each case up to 6 carbon atoms, or
represent a group of the formula -NR²R³, -NR⁴-L-R⁵ or -OR⁶,
R², R³ and R⁴ are identical or different and denote hydrogen or straight-chain or branched alkyl having up to 6 carbon atoms,
L denotes the -CO- or -SO₂- group,
R⁵ denotes straight-chain or branched alkyl having up to 6 carbon atoms or benzyl, or
denotes phenyl, which is optionally substituted by fluorine, chlorine, bromine, trifluoromethyl, trifluoromethoxy or hydroxyl or by straight-chain or branched alkyl or alkoxy having in each case up to 4 carbon atoms, and
R⁶ denotes straight-chain or branched alkyl or alkenyl having up to 6 carbon atoms, which are optionally substituted by cyclopropyl, cyclopentyl, cyclohexyl or phenyl,
or
A has one of the abovementioned meanings
and
B and D together form a radical of the formula 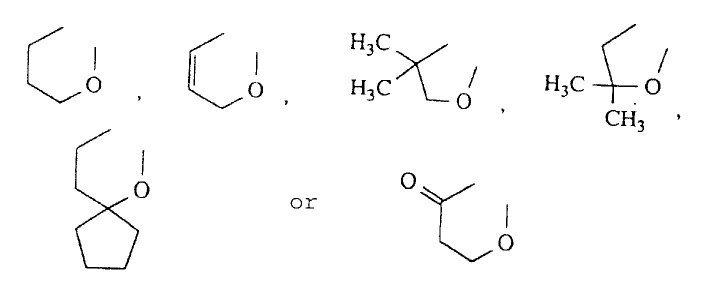
E represents a direct bond, or
represents straight-chain or branched alkylene, alkenylene or alkinylene having in each case up to 8 carbon atoms, which are optionally substituted by phenyl,
G represents phenyl, naphthyl, pyridyl, quinolyl, cyclopropyl, cyclobutyl, cyclopentyl, cyclohexyl, cycloheptyl, cyclooctyl or adamantyl, which are optionally substituted by up to 2 identical or different substituents from the group comprising hydroxyl, fluorine, chlorine, bromine, nitro, cyano, difluoromethyl, trifluoromethyl, difluoromethoxy and trifluoromethoxy and straight-chain or branched alkyl and alkoxy having in each case up to 6 carbon atoms, the latter optionally being substituted by phenyl or phenoxy, or
represents a radical of the formula 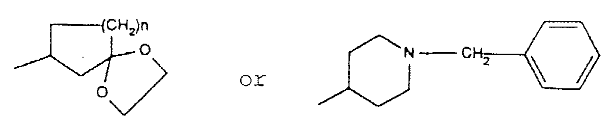 wherein
n denotes number 1 or 2,
and
R¹ represents hydrogen, represents the radical of the formula -E'-G',
wherein
E' and G' have the meaning given above for E and G and are identical to or different from these radicals,
if appropriate in an isomeric form,
and salts thereof,
N-[1-phenylethyl]-2-aminomethyl-chroman and N-[1-phenylethyl]-2-aminomethyl-8-methoxy-chroman being excepted.

2. Aminomethyl-chromans according to Claim 1, in which
A, B and D are identical or different and
represent hydrogen, fluorine, chlorine, bromine, cyano, trifluoromethyl, trifluoromethoxy or hydroxyl, or represent straight-chain or branched alkyl or alkenyl having in each case up to 4 carbon atoms, or represent a group of the formula -NR²R³ or -OR⁶,
wherein
R² and R³ are identical or different and denote hydrogen or straight-chain or branched alkyl having up to 4 carbon atoms and
R⁶ denotes straight-chain or branched alkyl or alkenyl having up to 4 carbon atoms, which are optionally substituted by cyclopropyl or phenyl,
or
A has one of the abovementioned meanings
and
B and D together form a radical of the formula 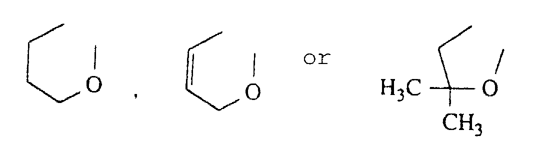
E represents a direct bond, or represents straight-chain or branched alkylene or alkenylene having in each case up to 7 carbon atoms, which are optionally substituted by phenyl,
G represents phenyl, naphthyl, adamantyl, cyclopropyl, cyclopentyl, cyclohexyl, cycloheptyl or cyclooctyl, which are optionally substituted by fluorine, chlorine, bromine, hydroxyl, difluoromethyl, trifluoromethyl, difluoromethoxy or trifluoromethoxy or by straight-chain or branched alkyl or alkoxy having in each case up to 4 carbon atoms, the latter optionally being substituted by phenyl or phenoxy, or
represents a radical of the formula 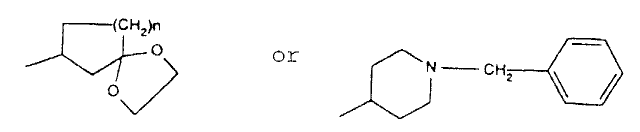
wherein
n denotes a number 1 or 2,
and
R¹ represents hydrogen or the radical of the formula
wherein
E' and G' have the meaning given above for E and G and are identical to or different from these radicals,
if appropriate in an isomeric form,
and salts thereof,
N-[1-phenylethyl]-2-aminomethyl-chroman and N-[1-phenylethyl]-2-aminomethyl-8-methoxy-chroman being excepted.

3. Aminomethyl-chromans according to Claim 1 for combating diseases.

4. Process for the preparation of aminomethyl-chromans according to Claim 1 and 2, characterised in that
[A1] compounds of the general formula (II) 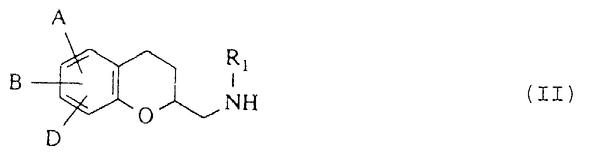 in which
A, B, D and R¹ have the abovementioned meaning,
are alkylated with compounds of the general formula (III)
M-E-G (III)
in which
M represents a typical leaving group, such as chlorine, bromine, iodine, tosylate or mesylate, or represents the group -OSO₂CF₃
and
E and G have the abovementioned meaning,
in inert solvents, if appropriate in the presence of a base and/or of a catalyst,
or
[A2] compounds of the general formula (IV) 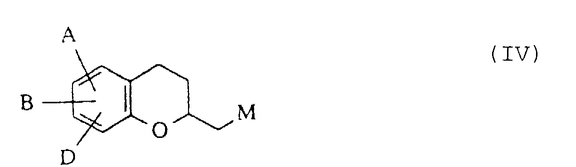 in which
A, B, D and M have the abovementioned meaning,
are alkylated with amines of the general formula (V) 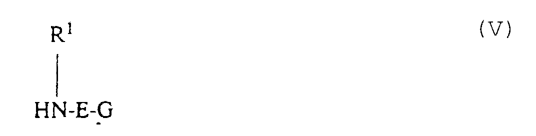
in which
R¹, E and G have the abovementioned meaning,
in inert solvents, if appropriate in the presence of a base and/or of a catalyst,
or
[B1] aldehydes of the general formula (VI) 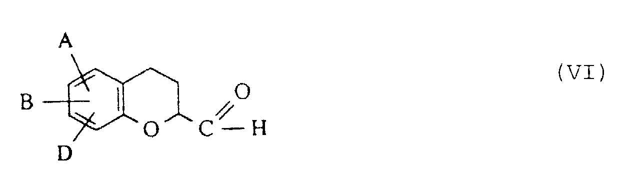 in which
A, B and D have the abovementioned meaning,
are alkylated reductively with amines of the general formula (V)
in inert solvents, if appropriate in the presence of auxiliaries,
or
[B2] in the case where E does not denote a direct bond, compounds of the general formula (II) are alkylated reductively either with aldehydes of the general formula (VII) 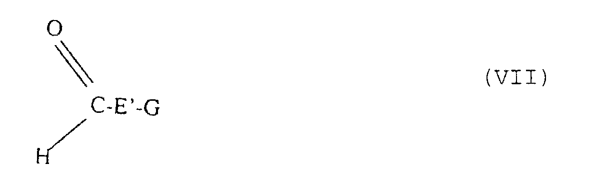 in which
G has the abovementioned meaning and
E' has the abovementioned meaning of E but is shorter by one -CH₂- group,
or, in the case where E represents a direct bond, with compounds of the general formula (VIIa)
O=G' (VIIa)
in which
G' has the abovementioned meaning of G, but does not represent aryl,
in inert solvents, if appropriate in the presence of auxiliaries,
or
[C] compounds of the general formulae (VIII) or (IX) 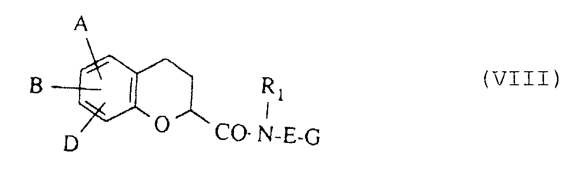 or 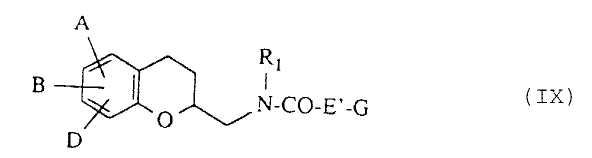 in which
A, B, D, E, E', G and R¹ have the abovementioned meaning,
are reduced by the customary method in inert solvents, if appropriate in the presence of auxiliaries,
or
[D] in the case where E represents 3 to 6 methylene groups,
compounds of the general formula (IIa) 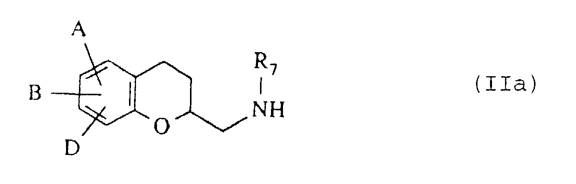 in which
A, B and D have the abovementioned meaning
and
R⁷ has the abovementioned meaning of R¹, but does not represent hydrogen,
are first reacted with either formaldehyde or formaldehyde derivatives and with compounds of the general formula (X)
E"-G (X)
in which
G has the abovementioned meaning
and
E" represents a radical of the formula HC/C-(CH₂)_{z},
wherein
z denotes the number 0, 1, 2 or 3,
in a reaction analogous to a Mannich reaction, and if appropriate hydrogenation by a customary method follows in a subsequent step,
and in the case where R¹ does not represent hydrogen, the product is either alkylated by the customary method or alkylated reductively with formaldehyde (R¹ = CH₃), as described above,
and in the case where R¹ denotes hydrogen, the amine function is first blocked with suitable amino-protective groups during individual process steps, if appropriate, and these are removed by a customary method, preferably by hydrogenolysis,
and if appropriate the substituents A, B, D and G are modified by a customary method.

5. Medicaments containing at least one aminomethylchroman according to Claim 1 and 2.

6. Medicaments according to Claim 5 for the treatment of diseases of the central nervous system.

7. Medicaments according to Claim 5 for the treatment of diseases which are characterised by disturbances of the serotoninergic system.

8. Use of aminomethyl-chromans according to Claim 1 and 2 for the preparation of medicaments.

9. Process for the preparation of medicaments according to Claim 5, characterised in that the aminomethyl-chromans are converted into a suitable administration form, if appropriate with customary auxiliaries and excipients.

## Revendications

1. Aminométhyl-chromanes de formule générale (i) 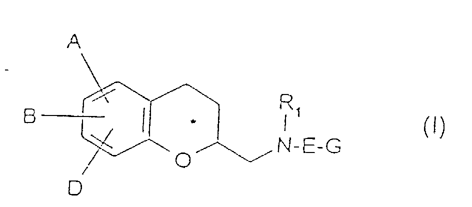 dans laquelle
A, B et D sont identiques ou différents et représentent l'hydrogène, le fluor, le chlore, le brome, un groupe cyano, trifïuorométhyle, difluorométhoxy, trifluorométhoxy ou hydroxy,
un groupe alkyle, alcényle, acyle ou alkoxycarbonyle linéaire ou ramifié ayant chacun jusqu'à 6 atomes de carbone,
un groupe de formule -NR²R³, -NR⁴-L-R⁵ ou -OR⁶,
R², R³ et R⁴ sont identiques ou différents et représentent l'hydrogène ou un groupe alkyle linéaire ou ramifié ayant jusqu'à 6 atomes de carbone,
L est le groupe -CO- ou -SO₂-
R⁵ est un groupe alkyle linéaire ou ramifié ayant jusqu'à 6 atomes de carbone ou un groupe benzyle, ou bien
un groupe phényle qui est substitué le cas échéant par du fluor, du chlore, du brome, un radical trifluorométhyle, trifluorométhoxy, hydroxy ou par un radical alkyle ou alkoxy linéaire ou ramifié dont chacun a jusqu'à 4 atomes de carbone,
R⁶ est un groupe alkyle ou alcényle linéaire ou ramifié ayant jusqu'à 6 atomes de carbone, ce groupe étant substitué le cas échéant par un radical cyclopropyle, cyclopentyle, cyclohexyle ou phényle,
ou bien
A a l'une des définitions indiquées ci-dessus
et
B et D forment conjointement un reste de formule : 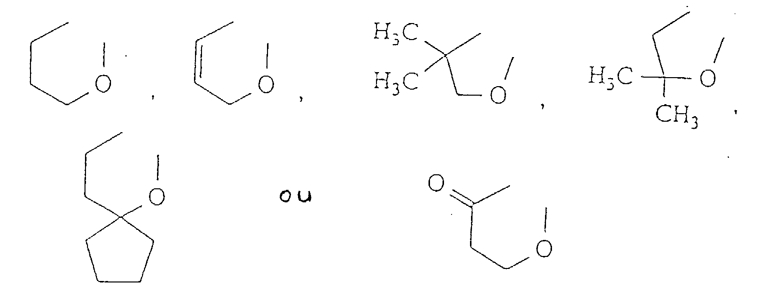
E est une liaison directe, ou bien
un groupe alkylène, alcénylène ou alcynylène linéaire ou ramifié ayant chacun jusqu'à 8 atomes de carbone et étant éventuellement substitué par un radical phényle,
G représente les groupe phényle, naphtyle, pyridyle, quinolyle, cyclopropyle, cyclobutyle, cyclopentyle, cyclohexyle, cycloheptyle, cyclo-octyle ou adamantyle qui sont substitués, le cas échéant jusqu'à 2 fois identiques ou différentes, par un radical hydroxy, fluoro, chloro, bromo, nitro, cyano, difluorométhyle, trifluorométhyle, difluorométhoxy, trifluorométhoxy ou par un radical alkyle ou un radical alkoxy linéaire ou ramifié ayant chacun jusqu'à 6 atomes de carbone, ces derniers radicaux portant éventuellement un substituant phényle ou phénoxy, ou bien
un reste de formule 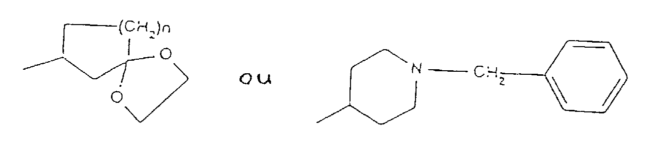
dans laquelle
n a la valeur 1 ou 2,
R¹ est l'hydrogène, le reste de formule -E'-G',
dans laquelle
E' et G' ont la définition indiquée ci-dessus pour E et G et y sont identiques ou en sont différents,
le cas échéant sous une forme isomère,
et leurs sels,
le N-[1-phényléthyl]-2-aminométhyl-chromane et le N-[1-phényléthyl]-2-aminométhyl-8-méthoxy-chromane étant exclus.

2. Aminométhyl-chromanes suivant la revendication
1, dans lesquels
A, B et D sont identiques ou différents et représentent l'hydrogène, le fluor, le chlore, le brome, un groupe cyano, trifluorométhyle, trifluorométhoxy ou hydroxy,
un groupe alkyle ou un groupe alcényle linéaire ou ramifié ayant chacun jusqu'à 4 atomes de carbone,
un groupe de formule -NR²R³ ou -OR⁶,
dans laquelle
R² et R³ sont identiques ou différents et représentent l'hydrogène ou un groupe alkyle linéaire ou ramifié ayant jusqu'à 4 atomes de carbone,
R⁶ est un groupe alkyle ou un groupe alcényle linéaire ou ramifié ayant jusqu'à 4 atomes de carbone, portant éventuellement un substituant cyclopropyle ou phényle,
ou bien
A a l'une des définitions indiquées ci-dessus
et
B et D forment ensemble un reste de formule : 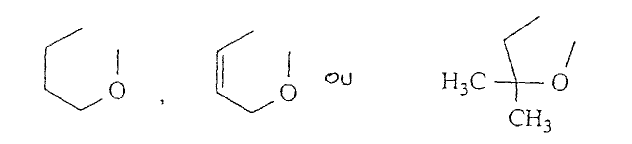
E est une liaison directe, ou bien
un groupe alkylène ou un groupe alcénylène linéaire ou ramifié ayant chacun jusqu'à 7 atomes de carbone et portant éventuellement un substituant phényle,
G représente les groupe phényle, naphtyle, adamantyle, cyclopropyle, cyclopentyle, cyclohexyle ou cyclo-octyle qui sont éventuellement substitués par du fluor, du chlore, du brome, un radical hydroxy, difluorométhyle, trifluorométhyle, difluorométhoxy, trifluorométhoxy ou par un radical alkyle ou un radical alkoxy linéaire ou ramifié ayant chacun jusqu'à 4 atomes de carbone, ces derniers radicaux portant éventuellement un substituant phényle ou phénoxy, ou bien
un reste de formule 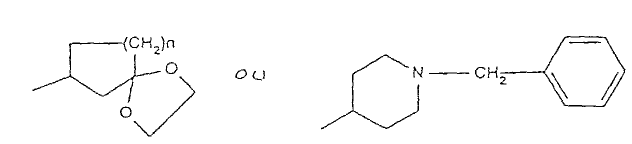
où
n représente le nombre 1 ou 2,
R¹ représente l'hydrogène ou le reste de formule -E'-G',
dans laquelle
E' et G' ont la définition indiquée ci-dessus pour E et G et y sont identiques ou en sont différents,
le cas échéant sous une forme isomère,
et leurs sels,
le N-[1-phényléthyl]-2-aminométhyl-chromane et le N-[1-phényléthyl]-2-aminométhyl-8-méthoxy-chromane étant exclus.

3. Aminométhyl-chromanes suivant la revendication 1, destinés à combattre des maladies.

4. Procédé de production diaminométhyl-chromanes suivant les revendications 1 et 2, caractérisé en ce que :
[A1] on alkyle des composés de formule générale (II) 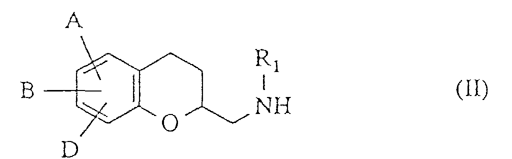 dans laquelle
A, B, D et R¹ ont la définition indiquée ci-dessus avec des composés de formule générale (III)
M-E-G (III)
dans laquelle
M est un groupe partant typique tel que chlore, brome, iode, tosylate, mésylate on le groupe -OSO₂CF₃,
et
E et G ont la définition indiquée ci-dessus,
dans des solvants inertes, éventuellement en présence d'une base et/ou d'un catalyseur,
ou bien
[A2] on alkyle des composés de formule générale (IV) 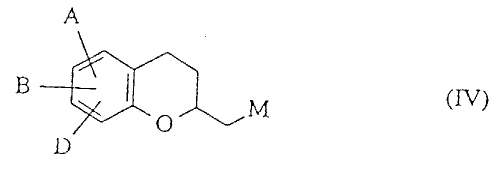 dans laquelle
A, B, D et M ont la définition indiquée ci-dessus, avec des amines de fonnule générale (V) 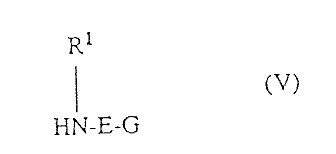 dans laquelle
R¹, E et G ont la définition indiquée ci-dessus, dans des solvants inertes, éventuellement en présence d'une base et/ou d'un catalyseur,
ou bien
[B1] on alkyle par voie de réduction des aldéhydes de formule générale (VI) 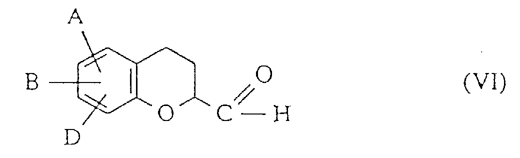 dans laquelle
A, B et D ont la définition indiquée ci-dessus, avec des amines de formule générale (V) dans des solvants inertes, éventuellement en présence de substances auxiliaires,
ou bien
[B2] au cas où E ne représente pas une liaison directe,
on alkyle par voie de réduction des composés de formule générale (II) ou bien avec des aldéhydes de formule générale (VII) 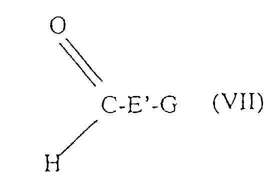 dans laquelle
G a la définition indiquée ci-dessus,
E' a la définition indiquée ci-dessus pour E, mais est raccourci d'un groupe -CH₂-,
ou bien au cas où E est une liaison directe, avec des composés de formule générale (VIIa)
O=G' (VIIa)
dans laquelle
G' a la définition indiquée ci-dessus pour G, excepté un groupe aryle,
dans des solvants inertes, éventuellement en présence de substances auxiliaires,
ou bien
[C] on réduit selon un mode opératoire classique des composés de formules générales (VIII) ou (IX) 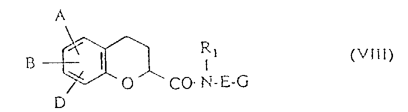 ou 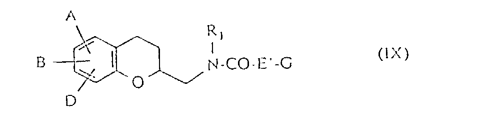 dans lesquelles
A, B, D, E, E', G et R¹ ont la définition indiquée ci-dessus,
dans des solvants inertes, éventuellement en présence de substances auxiliaires
ou bien
[D] au cas où E représente 3 à 6 groupes méthylène,
on fait réagir des composés de formule générale (IIa) 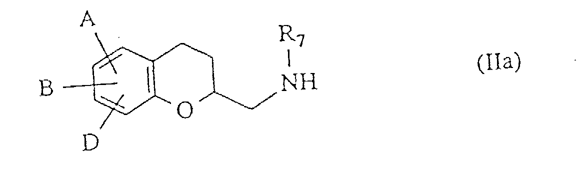 dans laquelle
A, B et D ont la définition indiquée ci-dessus,
et
R⁷ a la définition indiquée ci-dessus pour R¹, excepté l'hydrogène,
tout d'abord avec le formaldéhyde ou des dérivés de formaldéhyde et avec des composés de formule générale (X)
E"-G (X)
dans laquelle
G a la définition indiquée ci-dessus
et
E" représente un reste de formule HC≡C-(CH₂)_{z}, où
z représente le nombre 0, 1, 2 ou 3,
dans une réaction analogue à une réaction de Mannich et, le cas échéant, dans une étape subséquente, on effectue une hydrogénation selon un mode opératoire classique,
et au cas où R¹ ne représente pas de l'hydrogène, on effectue une alkylation selon un mode opératoire classique ou une
alkylation par voie de réduction avec le formaldéhyde (R¹=CH₃) comme décrit ci-dessus,
et au cas où R¹ représente l'hydrogène, on protège tout d'abord éventuellement la fonction amine pendant des étapes particulières du procédé avec des groupes convenant pour protéger la fonction amino et on élimine ces groupes par un mode opératoire classique, de préférence par hydrogénolyse,
et on modifie éventuellement les substituants A, B, D et G par un mode opératoire classique.

5. Médicament contenant au moins un aminométhylchromane suivant les revendications 1 et 2.

6. Médicament suivant la revendication 5, destiné au traitement de maladies du système nerveux central.

7. Médicament suivant la revendication 5, destiné au traitement de maladies qui sont caractérisées par des troubles du système sérotoninergique,

8. Utilisation d'aminométhyl-chromanes suivant les revendications 1 et 2 pour la préparation de médicaments.

9. Procédé de préparation de médicaments suivant la revendication 5, caractérisé en ce qu'on fait prendre une forme d'administration approriée aux aminométhyl-chromanes, le cas échéant avec des substances auxiliaires et des supports classiques.
